# EUROPEAN PATENT APPLICATION

(11) **EP 4 458 991 A1**
(43) Date of publication of application: **06.11.2024**
(21) Application number: 22914812.7
(22) Date of filing: 27.12.2022
(51) Int. Cl.: C12Q 1/70

(54) **PRIMER PROBE FOR DETECTION, PRIMER PROBE SET, AND APPLICATION THEREOF**

(30) Priority: 27.12.2021 CN 202111617233
(71) Applicant: Maccura Biotechnology Co., Ltd., Chengdu Sichuan 611731 (CN)
(72) Inventor: ZHAO, Yuhang, Chengdu, Sichuan 611731 (CN); HU, Waner, Chengdu, Sichuan 611731 (CN); ZHAN, Haomiao, Chengdu, Sichuan 611731 (CN); TANG, Fang, Chengdu, Sichuan 611731 (CN)
(74) Representative: Elzaburu S.L.P.
(86) International application number: PCT/CN2022/142454
(87) International publication number: WO 2023/125560

(57) **Abstract**

Disclosed are a primer probe for PCR detection, a primer probe set, and an application thereof. The primer probe and the primer probe set can simultaneously perform analysis in two dimensions of a fluorescent channel and a melting temperature in a single tube reaction. That is, by using a same fluorescent channel, different targets may be detected by means of melting temperature characteristics; or when different fluorescent channels are used, a target type detection of a product of the number of fluorescent channels and the melting temperature characteristic may be achieved. Moreover, a method for performing multiplex PCR detection by using the primer probe has the advantages of being low in fluorescence background, adjustable in melting temperature, good in inclusiveness, and low in cost, achieving single tube reaction, and being simple and convenient to operate, not easy to cause pollution, high in sensitivity, and wide in applicable range.

## Description

### Cross referencing of relevant applications

The present application claims the priority of Chinese Patent Application No. 202111617233.2 entitled "PRIMER PROBE FOR DETECTION, PRIMER PROBE SET, AND APPLICATION THEREOF" and filed on December 27, 2021, the content of which is incorporated herein by reference in its entirety.

### Field of the Invention

The invention relates to the technical field of molecular biology, in particular to a primer probe for PCR detection, a primer probe set, and an application thereof.

### Background of the Invention

Polymerase chain reaction (PCR) is a molecular biological technology that performs DNA replication via enzyme without using living organisms. The PCR is usually used in medical and biological research laboratories to undertake a variety of tasks, such as diagnosis of infectious diseases, gene cloning, phenotypic identification of laboratory animals, research on transcriptomes, detection of genetic diseases, identification of gene fingerprints, paternity test, etc. Since the PCR has an incomparable replication ability and accuracy, it is considered by the molecular biologists as a preferred method for nucleic acid detection. In the late 1990s, a real-time fluorescence quantitative PCR (qPCR) technology and related products launched by ABI in the USA further developed PCR to be a highly sensitive, highly specific and accurately quantitative nucleic acid sequence analysis technology.

At present, a primer probe design method that is widely used in qPCR platforms is mainly a TaqMan hydrolysis probe method, a working principle of which is mainly characterized by using an oligonucleotide probe that can specifically bind with a template and is labelled with a fluorescence group (donor) and a quencher group (receptor) at two ends thereof, and by designing a specific PCR primer upstream and downstream of the probe respectively. Before the start of PCR reaction, due to a principle of fluorescence resonance energy transfer (FRET), a fluorescence signal emitted by the fluorescence group at one end of the TaqMan probe is absorbed by the quencher group at the other end, such that the fluorescence signal cannot be detected by an instrument; but after the start of PCR amplification, the TaqMan probe specifically binds with the template, and when a DNA polymerase (Taqase) extends to a site at which the probe binds to the template, the TaqMan probe will be cleaved due to 5-3' exonuclease activity of the DNA polymerase (Taqase), such that the fluorescence group labelled on the probe will be far away from the quencher group, and a FRET structure cannot be formed any more, and thus a signal emitted by the fluorescence group can be detected by the instrument. However, in a same reaction system, in order to achieve PCR detection of multiplex targets, it is required to arrange multiple TaqMan hydrolysis probes labeled with fluorescence groups of different wavelengths, and it can achieve detection of only 4-6-plex different targets at most depending on the number of fluorescent channels in a detection instrument.

In order to achieve an ultra-multiplex PCR detection, in the prior art, the PCR detection technology is usually combined with other methods, for example, the PCR is combined with a hybrid chip to perform detection of multiplex targets with different amplification product lengths. For example, a document CN107090519A discloses a detection kit for common respiratory pathogens combining multiplex RT-PCR with a gene chip and detection method thereof, in the method, by utilizing the principle of nucleic acid molecule hybridization, single-stranded probes targeting individual targets are arranged and fixed on the gene chip in a specific order to form a probe array, and then multiplex PCR products to be detected are allowed to be hybridized with it, such that target amplification products are hybridized with the probes on the chip and emit fluorescence signals. Although 20 types of respiratory pathogens can be simultaneously detected by this method, the gene chip is complex in preparation process, expensive, and not conducive to clinical promotion. Moreover, the method is cumbersome in procedures, requires multiple steps of uncovering and cleaning, and may easily cause contamination and result in a false positive result. For another example, a document CN103074450B discloses a kit for simultaneous detection of thirty types of pathogenic bacteria causing diarrhea and a detection method thereof. The method combines the PCR amplification and capillary electrophoresis, PCR amplification products are taken out and subjected to capillary electrophoresis analysis; and the obtained spectrum is compared with a standard spectrum to determine the types of pathogenic bacteria causing diarrhea. In addition to the use of a PCR amplification detection equipment, this method further requires the use of a capillary electrophoresis equipment for product analysis, such that a detection cost is greatly increased, thereby being not conducive to clinical promotion and application.

Therefore, there has been an urgent clinical need for a method that can perform multiplex molecular detection, so as to quickly, simply and conveniently detect multiple types of clinically common targets such as bacteria, viruses, gene mutations, etc. at a low cost, such that clinical units, including primary hospitals, can quickly carry out a common target detection (such as a pathogenic microorganism detection), which may be assisted with other detection and definite diagnostic means, thereby quickly providing diagnostic evidence and medication schemes for clinical practice, reducing mental and economic burden of patients, and achieving an objective of precision medicine.

### Summary of the Invention

The present invention is directed at the deficiencies of methods for detecting and differentiating multiple different targets in a single tube reaction in the prior art, and provides a primer probe for PCR detection and a primer probe set. A method of detecting and differentiating multiple different targets by using the primer probe or the primer probe set can effectively avoid false positives caused by primer dimers.

For this purpose, in a first aspect, the present invention provides a primer probe for PCR detection, comprising a first primer, a probe, and a second primer; wherein,
the first primer comprises a target sequence binding region 1, which is a sequence that can specifically bind with a target sequence in a paired manner;
the probe (P) is a sequence that does not bind with any target sequence in a paired manner, and comprises a probe signal detection region (H); and the probe (P) is labeled with a detection label; the second primer comprises a primer signal detection region (h) and a target sequence binding region 2; wherein, the target sequence binding region 2 is a sequence that can specifically bind with the target sequence in a paired manner, the primer signal detection region (h) is a sequence that does not bind with any target sequence in a paired manner, and a complementary sequence (h') of the primer signal detection region (h) specifically binds with the probe signal detection region (H) of the probe (P) in a paired manner; and the target sequence binding region 2 is located at 3' end of the primer signal detection region (h);
and the first primer and the second primer specifically bind with the target respectively to produce a pre-amplification product, and the pre-amplification product contains one single-stranded pre-amplification product having the complementary sequence (h') of the primer signal detection region (h), the complementary sequence (h') in the single-stranded pre-amplification product specifically binds with the probe (P) and extends by ≥ 0 bases to form a double-stranded product, and the formation of the double-stranded product causes the probe to produce a detectable signal change.

In the present invention, the first primer is a forward primer (F), and the second primer is a reverse primer (R); or
the first primer is a reverse primer (R), and the second primer is a forward primer (F).

In some embodiments of the present invention, the first primer is a forward primer (F), and the second primer is a reverse primer (R).

In the present invention, the complementary sequence (h') of the primer signal detection region (h) is a forward-complementary sequence (h') of the primer signal detection region (h) or a reverse-complementary sequence (h') of the primer signal detection region (h).

In the present invention, the "specifically bind in a paired manner" or "specifically bind" refers to the binding of two single-stranded nucleic acid molecules with complementary sequences through base complementary pairing under certain conditions (suitable temperature and ion strength, etc.). It is well known for those skilled in the art that, two single-stranded nucleic acid molecules may specifically bind although the base pairing is not completely complementary, and in the case that there are few mismatching bases, they can also specifically bind with each other.

In some embodiments of the present invention, the complementary sequence (h') of the primer signal detection region (h) is the reverse-complementary sequence (h') of the primer signal detection region (h).

In the present invention, the complementary sequence (h') of the primer signal detection region (h) binds with the probe signal detection region (H) of the probe (P) in a forward-complementary paired manner or in a reverse-complementary paired manner.

In some embodiments of the present invention, the complementary sequence (h') of the primer signal detection region (h) binds with the probe signal detection region (H) of the probe (P) in a reverse-complementary paired manner.

In some embodiments of the present invention, the complementary sequence (h') of the primer signal detection region (h) is the reverse-complementary sequence (h') of the primer signal detection region (h).

In some embodiments of the present invention, the reverse-complementary sequence (h') of the primer signal detection region (h) binds with the probe signal detection region (H) of the probe (P) in a forward-complementary paired manner or in a reverse-complementary paired manner.

In some embodiments of the present invention, the primer signal detection region (h) and the probe signal detection region (H) of the probe (P) have a partially or completely identical sequence.

In some embodiments of the present invention, the double-stranded product is a secondary amplification double-stranded product.

In some embodiments of the present invention, the primer probe comprises a forward primer (F), a probe (P), and a reverse primer (R); wherein,
the primer (F) comprises a target sequence binding region 1, which is a sequence that can specifically bind with the target sequence in a paired manner;
the probe (P) is a sequence that does not bind with any target sequence in a paired manner, and comprises a probe signal detection region (H); and the probe (P) is labeled with a detection group; the primer (R) sequentially comprises a primer signal detection region (h) and a target sequence binding region 2 from 5' end to 3' end; wherein, the target sequence binding region 2 is a sequence that can specifically bind with the target sequence in a paired manner, and the primer signal detection region (h) is a sequence that does not bind with any target sequence in a paired manner and has a sequence partially or completely identical to that of the probe signal detection region (H) of the probe (P);
the forward primer (F) and the reverse primer (R) specifically bind with the target respectively to produce a pre-amplification product, the pre-amplification product contains one single-stranded pre-amplification product having a reverse-complementary sequence (h') of the primer signal detection region (h), the reverse-complementary sequence (h') of the single-stranded pre-amplification product specifically binds with the probe (P) and then extends to form a secondary amplification double-stranded product, and the formation of the secondary amplification double-stranded product causes the probe to produce a detectable signal change.

In some embodiments of the present invention, the first primer sequentially comprises a probe anchoring region (A) and a target sequence binding region 1 from 5' end to 3' end; wherein, the target sequence binding region 1 is a sequence that can specifically bind with a target sequence in a paired manner, and the probe anchoring region (A) is a sequence that does not bind with any target sequence in a paired manner; and
the probe (P) is a sequence that does not bind with any target sequence in a paired manner, and comprises a primer anchoring region (A') and a probe signal detection region (H); and the primer anchoring region (A') is a sequence complementary with the probe anchoring region (A) of the first primer.

In the present invention, the primer anchoring region (A') is a sequence that is forward complementary or reverse complementary with the probe anchoring region (A) of the first primer. In some embodiments of the present invention, the probe (P) comprises 20-100 bases.

In some embodiments of the present invention, the first primer further comprises a probe anchoring region (A); wherein the probe anchoring region (A) is a sequence that does not bind with any target sequence in a paired manner, and is located at 5' end of the target sequence binding region 1; and the probe (P) further comprises a primer anchoring region (A'); and the primer anchoring region (A') is a sequence complementary with the probe anchoring region (A) of the first primer.

In some embodiments of the present invention, the forward primer (F) sequentially comprises a probe anchoring region (A) and a target sequence binding region 1 from 5' end to 3' end; wherein, the target sequence binding region 1 is a sequence that can specifically bind with a target sequence in a paired manner, and the probe anchoring region (A) is a sequence that does not bind with any target sequence in a paired manner; the probe (P) is a sequence that does not bind with any target sequence in a paired manner, and comprises a primer anchoring region (A') and a probe signal detection region (H); and the primer anchoring region (A') is a sequence complementary with the probe anchoring region (A) of the forward primer (F).

The expression that "the primer anchoring region (A') is complementary with the probe anchoring region (A)" refers to that the two may be forward complementary or reverse complementary, as long as they can be complementary pairing.

In the present invention, the term "sequentially comprise" does not mean that there is no base spacing, and there may be 0-20 bases spaced between adjacent sequences.

The above expression "the forward primer (F) sequentially comprises a probe anchoring region (A) and a target sequence binding region 1 from 5' end to 3' end" does not mean that there is no base spacing between the probe anchoring region (A) and the target sequence binding region 1, and there may be 0-20 bases spaced between the probe anchoring region (A) and the target sequence binding region 1.

In some embodiments of the present invention, there are 0-20 bases spaced between the primer signal detection region (h) and the target sequence binding region 2 in the second primer.

In some embodiments of the present invention, there are 0-20 bases spaced between the primer signal detection region (h) and the target sequence binding region 2 of the reverse primer (R).

In some embodiments of the present invention, the detection label is a detection group.

In some embodiments of the present invention, the detection label comprises a first detection group and a second detection group, and the first detection group and the second detection group generate a signal change via a distance change; preferably, the first detection group is spaced from the second detection group by 3-250 angstroms; preferably, by 3-201 angstroms; more preferably, by 3-140 angstroms; and further preferably, the first detection group is a fluorescence reporter group, and the second detection group is a quencher group or other labeling groups that can produce a signal change with the first detection group via fluorescence resonance energy transfer.

In the present invention, the positions of the first detection group and the second detection group on the probe allow that the reverse-complementary sequence (h') of the single-stranded pre-amplification product specifically binds with the probe (P) and extends by ≥ 0 bases to form a double-stranded product; and the formation of the double-stranded product can cause a change of the positions of the first detection group and the second detection group, it can cause the probe to produce a detectable signal change. The positions of the first detection group and the second detection group may be interchanged. For different situations, the positions of the first detection group and the second detection group on the probe may be differently defined to achieve a effect of reducing fluorescence background. For example, the probe (P) sequentially comprises a primer anchoring region (A') and a probe signal detection region (H) from 5' end to 3' end; and the positions of the first detection group and the second detection group on the probe (P) may be labeled between the primer anchoring region (A') and the probe signal detection region (H), respectively.

In some embodiments of the present invention, the positions of the first detection group and the second detection group on the probe allow that the reverse-complementary sequence (h') of the single-stranded pre-amplification product specifically binds with the probe (P) and extends to form a secondary amplification double-stranded product; and the formation of the secondary amplification double-stranded product causes the probe to produce a detectable signal change. For example, at least one detection group is labeled at 5' end of the probe signal detection region (H) of the probe, or at least one detection group is labeled within the probe signal detection region (H) of the probe.

In some embodiments, the probe (P) comprises 1-2 primer anchoring regions (A') and 1-2 probe signal detection regions (H).

In some embodiments of the present invention, the probe (P) is any one structure of 1) to 4);
1) the probe (P) comprising one primer anchoring region (A') and one probe signal detection region (H);
2) the probe (P) compring one probe signal detection region (H) and two primer anchoring regions (A'), wherein the two primer anchoring regions (A') are located on two sides of the probe signal detection region (H), respectively, and the two primer anchoring regions (A') are different in sequence;
3) the probe (P) comprising one primer anchoring region (A') and two probe signal detection regions (H), wherein the two probe signal detection regions (H) are located on two sides of the primer anchoring region (A'), and the two probe signal detection regions (H) are different in sequence; and
4) the probe (P) comprising two primer anchoring regions (A') and two probe signal detection regions (H), wherein the primer anchoring regions (A') and the probe signal detection regions (H) are arranged at intervals, the two primer anchoring regions (A') are different in sequence, and the two probe signal detection regions (H) are different in sequence.

In the above embodiments of the present invention, a relationship between the primer anchoring region (A') and the probe signal detection region (H) on the probe (P) is as follows:
① only the probe signal detection region (H) is contained, and the primer anchoring region (A') is not contained; correspondingly, the upstream primer (F) does not contain the probe anchoring region (A);
(2) one probe signal detection region (H) and one primer anchoring region (A') are contained: the positions of the two may be interchanged (A'-H or H-A'), there may or may not be a base spacing therebetween;
(3) two probe signal detection regions (H) and one primer anchoring region (A') are contained: the probe signal detection regions (H) are distributed on two sides of the primer anchoring region (A'-H-A'); and there may or may not be a base spacing between the probe signal detection regions (H) and the one primer anchoring region (A');
④ two primer anchoring regions (A') and two probe signal detection regions (H) are contained: the primer anchoring regions (A') and the probe signal detection regions (H) are arranged at intervals (H-A'-H-A' or A'-H-A'-H); and there may or may not be a base spacing between the probe signal detection regions (H) and the primer anchoring regions (A').

In some embodiments of the present invention, the primer anchoring region (A') of the probe (P) is located at 5' end of the probe signal detection region (H); and preferably, the detection groups on the probe (P) are labeled between the primer anchoring region (A') and the probe signal detection region (H).

The expression "the probe (P) sequentially comprises a primer anchoring region (A') and a probe signal detection region (H) from 5' end to 3' end" does not mean that there is no base spacing between the primer anchoring region (A') and the probe signal detection region (H).

In some other embodiments of the present invention, the primer anchoring region (A') of the probe (P) is located at 5' end of the probe signal detection region (H); and preferably, the detection groups on the probe (P) are labeled at 5' end of the probe signal detection region (H).

In some specific embodiments of the present invention, the probe (P) in the primer probe is a freely designed sequence that does not bind with any target sequence in a paired manner, and the probe (P) sequentially comprises a primer anchoring region (A') and a probe signal detection region (H) from 5' end to 3' end; a sequence of the primer anchoring region (A') is complementary with a sequence of the probe anchoring region (A) of the first primer; and a second detection group is labeled at a base position 3-140 angstroms apart from a first detection group, wherein the first detection group may be a fluorescence reporter group, and the second detection group may be a quencher group or other labeling groups that can produce a signal change with the first detection group via fluorescence resonance energy transfer (FRET).

In some specific embodiments of the present invention, the probe (P) in the primer probe is a freely designed sequence that does not bind with any target sequence in a paired manner, and the probe (P) sequentially comprises a probe signal detection region (H) and a primer anchoring region (A') from 5' end to 3' end; a sequence of the primer anchoring region (A') is complementary with a sequence of the probe anchoring region (A) of the first primer; and a second detection group is labeled at a base position 3-140 angstroms apart from a first detection group, wherein the first detection group may be a fluorescence reporter group, and the second detection group may be a quencher group or other labeling groups that can produce a signal change with the first detection group via fluorescence resonance energy transfer (FRET).

In some specific embodiments of the present invention, the probe (P) in the primer probe is a freely designed sequence that does not bind with any target sequence in a paired manner, and the probe (P) comprises a primer anchoring region (A') and two probe signal detection regions (H); the probe (P) sequentially comprises a probe signal detection region (H1), the primer anchoring region (A'), and a probe signal detection region (H2) from 5' end to 3' end; a sequence of the primer anchoring region (A') is complementary with a sequence of the probe anchoring region (A) of a first primer; and a second detection group is labeled at a base position 3-140 angstroms apart from a first detection group, wherein the first detection group may be a fluorescence reporter group, and the second detection group may be a quencher group or other labeling groups that can produce a signal change with the first detection group via fluorescence resonance energy transfer (FRET).

In some embodiments of the present invention, a blocking region is contained at 3' end of the probe (P).

In the present invention, the blocking region is a portion that is used to prevent extension of the nucleic acid strand through DNA polymerase, thereby blocking strand extension during, for example, a PCR process. The blocking region may function as follows: 3'OH is labeled by 3'-Spacer C3, 3'-Phosphat, 3'-ddC, 3'-InvertedEnd, etc., such that the 3' OH is blocked, so as to prevent its extension reaction. The blocking region may also be a polymerase enzyme blocking group, which is a group that has a function property of blocking further enlongation of a polymer. The blocking group may be any chemical group that can connect to a nucleotide, which allows 5' end of a labeled nucleotide to connect to 3' end of another nucleotide in a DNA strand, but does not allow a nucleotide to connect to 3' hydroxyl group of the labeled nucleotide. Properly, the absence of OH group at 3' position will prevent further extension by means of polymerase activity. In some embodiments, the blocking group is selected from acetyl, CH3, glycyl, lencyl, and alanyl groups. In some other embodiments, the blocking group may be in a form of a dipeptide or tripeptide.

In some embodiments of the present invention, the second primer contains an extention blocking region (M) at 5' end thereof.

In some embodiments of the present invention, the pre-amplification product contains one single-stranded pre-amplification product having a reverse complementary sequence (h') of the primer signal detection region (h) and a complementary sequence (M') of the extension blocking region (M); and the complementary sequence (M') of the extention blocking region (M) is a sequence that does not specifically bind with any part of the probe (P) in a paired manner.

In the present invention, the complementary sequence (M') of the extention blocking region (M) is a forward-complementary sequence (M') of the extention blocking region (M) or a reverse-complementary sequence (M') of the extention blocking region (M).

In some embodiments of the present invention, the complementary sequence (M') of the extention blocking region (M) is the reverse-complementary sequence (M') of the extention blocking region (M).

In the present invention, the complementary sequence (M') of the extention blocking region (M) is a sequence that does not bind with any part of the probe (P) in a forward-complementary paired manner or in a reverse-complementary paired manner.

In some embodiments of the present invention, the complementary sequence (M') of the extention blocking region (M) is a sequence that does not bind with any part of the probe (P) in a reverse-complementary paired manner.

In some embodiments of the present invention, the complementary sequence (M') of the extention blocking region (M) is the reverse-complementary sequence (M') of the extention blocking region (M); and the complementary sequence (M') of the extention blocking region (M) is a sequence that does not bind with any part of the probe (P) in a forward-complementary paired manner or in a reverse-complementary paired manner.

In some specific embodiments of the present invention, the first primer sequentially comprises a probe anchoring region (A) and a target sequence binding region 1 from 5' end to 3' end; wherein, the probe anchoring region (A) is a sequence that does not bind with any target sequence in a paired manner; and there may or may not be a base spacing between the probe anchoring region (A) and the target sequence binding region 1;
the probe (P) is a sequence that does not bind with any target sequence in a paired manner, and comprises a primer anchoring region (A') and a probe signal detection region (H); the primer anchoring region (A') is a sequence complementary with the probe anchoring region (A) of the first primer; and there may or may not be a base spacing between the primer anchoring region (A') and the probe signal detection region (H);
the second primer sequentially comprises an extension blocking region (M), a primer signal detection region (h), and a target sequence binding region 2 from 5' end to 3' end; and there may or may not be a base spacing between the extention blocking region (M) and the primer signal detection region (h), as well as between the primer signal detection region (h) and the target sequence binding region 2; and
the pre-amplification product contains a single-stranded pre-amplification product comprising the probe anchoring region (A), the target sequence, a reverse-complementary sequence (h') of the primer signal detection region (h), and a reverse-complementary sequence (M') of the extension blocking region (M) from 5' end to 3' end.

In some embodiments of the present invention, a Tm value of the primer signal detection region (h) is simultaneously lower than a Tm value of the target sequence binding region 1 in the first primer and a Tm value of the target sequence binding region 2 in the second primer.

The Tm value of the primer signal detection region (h) is simultaneously lower than the Tm value of the target sequence binding region 1 in the first primer and the Tm value of the target sequence binding region 2 in the second primer.

In some embodiments of the present invention, the Tm value of the primer signal detection region (h) is simultaneously lower than a Tm value of the target sequence binding region 1 in the forward primer (F) and a Tm value of the target sequence binding region 2 in the reverse primer (R).

In some other embodiments of the present invention, a Tm value of the primer anchoring region (A') is higher than the Tm value of the primer signal detection region (h).

In the present invention, by limiting the Tm value of the primer signal detection region (h) and the Tm value of the primer anchoring region (A') within the aforementioned range, the generation of primer dimers during amplification may be effectively avoided. It is noted that, even if a dimer is generated during the amplification process, a signal of the dimer may be differentiated by a melting curve; and a signal collection is perfomed at a higher melting temperature, such that the signal of the dimer will not be collected. A specific principle lies in that: the signal change generated by the double-stranded product formed by specific binding of the reverse-complementary sequence (h') of the single-stranded pre-amplification product and the probe (P), is different from the signal change generated by the secondary amplification double-stranded product formed by specific binding and extending of the reverse-complementary sequence (h') of the single-stranded pre-amplification product and the probe (P). A difference in signal change means a difference in signal strength and/or a difference in melting temperature.

In some embodiments of the present invention, there are 0-20 bases spaced between the probe anchoring region (A) and the target sequence binding region 1 in the first primer.

In some embodiments of the present invention, there are 0-20 bases spaced between the probe anchoring region (A) and the target sequence binding region 1 of the forward primer (F).

In some embodiments of the present invention, the forward primer (F) and the reverse primer (R) each independently have a length of 15-80 bases; preferably, the target sequence binding region 1 and the target sequence binding region 2 each independently have a Tm value of 40°C-80°C, and a GC content of 20%-80%; and the primer signal detection region (h) has a length of 5-65 bases, a Tm value of 30-80°C, and a GC content of 20-80%.

In some other embodiments of the present invention, the probe (P) has a length of 20-100 bases; and preferably, the probe signal detection region (H) has a length of 5-65 bases, a Tm value of 30-85°C, and a GC content of 20-80%.

In some embodiments of the present invention, the probe anchoring region (A) has a length of 6-35 bases, a Tm value of 40°C-80°C, and a GC content of 30%-80%.

In some other embodiments of the present invention, the primer anchoring region (A') has a length of 6-35 bases, a Tm value of 40°C-85°C, and a GC content of 20%-80%; and preferably, the Tm value of the primer anchoring region (A') is higher than the Tm value of the primer signal detection region (h).

In the present invention, the Tm value of the primer anchoring region (A') is allowed to be higher than the Tm value of the primer signal detection region (h), such that the binding of the reverse-complementary sequence (h') of the primer signal detection region to the probe needs the help of the probe anchoring region (A).

In some specific embodiments of the present invention, the first primer in the primer probe set sequentially comprises a probe anchoring region (A) and a target sequence binding region 1 from 5' end to 3' end.

In some specific embodiments of the present invention, the forward primer (F) in the primer probe sequentially comprises a probe anchoring region (A) and a target sequence binding region 1 from 5' end to 3' end; wherein, the target sequence binding region 1 is a sequence that can specifically bind with a target sequence in a paired manner, and the probe anchoring region (A) is a freely designed sequence that does not bind with any target sequence in a paired manner and has a sequence complementary with the primer anchoring region (A') of the probe (P); and there may be 0-20 bases spaced between the probe anchoring region (A) and the target sequence binding region 1.

In some other specific embodiments of the present invention, the probe (P) in the primer probe is a freely designed sequence that does not bind with any target sequence in a paired manner, and comprises a primer anchoring region (A'), and the other part thereof except for the primer anchoring region (A') is the probe signal detection region (H); a sequence of the primer anchoring region (A') is complementary with a sequence of the probe anchoring region (A) of the forward primer (F); and the second detection group is modifed at a base position 5-25 bases apart from the first detection group, and both of the first detection group and the second detection group are located on a same side of the primer anchoring region (A'), wherein the first detection group may be a fluorescence reporter group, and the second detection group may be a quencher group or other labeling groups that can produce a signal change with the first detection group via fluorescence resonance energy transfer (FRET).

In some specific embodiments of the present invention, the second primer in the primer probe sequentially comprises a primer signal detection region (h) and a target sequence binding region 2 from 5' end to 3' end.

In some specific embodiments of the present invention, the reverse primer (R) in the primer probe sequentially comprises a primer signal detection region (h) and a target sequence binding region 2 from 5' end to 3' end, wherein the target sequence binding region 2 is a sequence that can specifically bind with a target sequence in a paired manner; the primer signal detection region (h) is a freely designed sequence that does not bind with any target sequence in a paired manner, and has a sequence partially or completely identical to that of the probe signal detection region (H) of the probe (P); and there may be 0-20 bases spaced between the primer signal detection region (h) and the target sequence binding region 2.

It is noted that, the sequence structures of the forward primer and the reverse primer in the present invention may be interchanged. For example, the forward primer (F) comprises a primer signal detection region (h) and a target sequence binding region, while the reverse primer comprises a target sequence binding region; or, the forward primer (F) comprises a primer signal detection region (h) and a target sequence binding region, while the reverse primer (R) comprises a probe anchoring region (A) and a target sequence binding region.

A principle of performing PCR detection to a sample to be detected by using the above primer probe of the present invention is as follows:
During PCR amplification, the forward primer (F) and reverse primer (R) in the primer probe specifically bind with a target sequence in the sample to be detected respectively and extend to produce a double-stranded pre-amplification product, wherein one single-stranded pre-amplification product (S1) comprises the probe anchoring region (A), the target sequence, and the reverse-complementary sequence (h') of the primer signal detection region from 5' end to 3' end; after the double-stranded pre-amplification product is producted, the probe signal detection region (H) of the probe (P) in the primer probe is complementarily paired with the reverse-complementary sequence (h') of the primer signal detection region at 3' end of the single-stranded pre-amplification product (S1), and the primer anchoring region (A') of the probe (P) is complementarily paired with the probe anchoring region (A) at 5' end of the single-stranded pre-amplification product (S1), and the 3' end of the single-stranded pre-amplification product (S1) may continue to extend to the 5' end of the probe (P) to obtain a partially or completely reverse-complementary sequence of the probe (P), that is, a secondary amplification in which the single-stranded pre-amplification product (S1) is used as a primer and the probe (P) is used as a template is completed, to obtain a secondary amplification double-stranded product formed with the probe (P). At this time, the probe (P) is changed from a single-stranded state to a double-stranded state, and a distance between the first detection group and the second detection group is changed, thereby generating a signal that may be detected by an instrument;
and after the PCR amplification is completed, a melting curve analysis is performed, wherein, during a process of temperature change, the amplification double-stranded product formed with the probe (P) will melt at a certain temperature, at this time, the probe (P) with the first detection group and the second detection group will be changed from the double-stranded state to the single-stranded state, so as to cause a change in the distance between the detection groups, thereby generating a signal that may be detected by the instrument. The signals detected by the instrument are shown as a melting curve with the change of the temperature, and when a Tm value at a peak position of the melting curve is within a range of a characteristic Tm value of the target in the sample to be detected in a specific detection channel, it indicates that there is a target contained in the sample to be detected. The specific detection channel is a fluorescent channel corresponding to the first detection group on the probe (P), and the characteristic Tm value is a melting temperature of the secondary amplification double-stranded product formed with the probe (P).

In some preferred specific embodiments of the present invention, the primer probe comprises:
one first primer, sequentially comprising a probe anchoring region (A) and a target sequence binding region 1 from 5' end to 3' end; wherein, the target sequence binding region 1 is a sequence that can specifically bind with a target sequence in a paired manner, and the probe anchoring region (A) is a freely designed sequence that does not bind with any target sequence in a paired manner, and has a sequence complementary (reverse-complementary or forward-complementary) with 6-35 bases of a primer anchoring region (A') of a probe (P); and there may be 0-20 bases spaced between the probe anchoring region (A) and the target sequence binding region 1;
one probe (P), being a freely designed sequence that does not bind with any target sequence in a paired manner, and sequentially comprising a primer anchoring region (A') and a probe signal detection region (H) from 5' end to 3' end; wherein a sequence of the primer anchoring region (A') is complementary with a sequence of the probe anchoring region (A) of the first primer; and a second detection group is labeled at a base position 3-140 angstroms apart from a first detection group, wherein the first detection group may be a fluorescence reporter group, and the second detection group may be a quencher group or other labeling groups that can produce a signal change with the first detection group via fluorescence resonance energy transfer (FRET); and
one second primer, sequentially comprising a primer signal detection region (h) and a target sequence binding region 2 from 5' end to 3' end, wherein the target sequence binding region 2 is a sequence that can specifically bind with the target sequence in a paired manner; the primer signal detection region (h) in the second primer is a freely designed sequence that does not bind with any target sequence in a paired manner, and has a sequence partially or completely identical to that of the probe signal detection region (H) of the probe (P); and there may be 0-20 bases spaced between the primer signal detection region (h) and the target sequence binding region 2.

The pre-amplification product contains one single-stranded pre-amplification product sequentially comprising the probe anchoring region (A), the target sequence, and a reverse-complementary sequence (h') of the primer signal detection region (h) from 5' end to 3' end. The reverse-complementary sequence (h') in the single-stranded pre-amplification product dose not specifically bind the probe (P) at 5' end of the probe (P).

In the present invention, a principle of performing PCR detection to a sample to be detected by using the above primer probe is as follows:
During PCR amplification, the first primer and second primer in the primer probe specifically bind with the target sequence respectively and are subjected to amplification to produce a double-stranded pre-amplification product, wherein one single-stranded pre-amplification product (S1) comprises the probe anchoring region (A), the target sequence, and a reverse-complementary sequence (h') of the primer signal detection region from 5' end to 3' end.

After the double-stranded pre-amplification product is produced, the probe signal detection region (H) of the probe (P) is complementarily paired with the reverse-complementary sequence (h') of the primer signal detection region at the 3' end of the single-stranded pre-amplification product (S1), and the primer anchoring region (A') of the probe (P) is complementarily paired with the probe anchoring region (A) at 5' end of the single-stranded pre-amplification product (S1), and the 3' end of the single-stranded pre-amplification product (S1) may continue to extend, to obtain a partially or completely reverse-complementary sequence of the probe (P), that is, an amplification in which the single-stranded pre-amplification product (S1) is used as a primer and the probe (P) is used as a template is completed, to obtain a secondary amplification double-stranded product formed with the probe (P). At this time, a partially or completely double-stranded state is formed by the probe (P) and the single-stranded pre-amplification product (S1), and a distance between the first detection group and the second detection group is changed, thereby generating a signal that may be detected by an instrument;

After the PCR amplification is completed, the resultant is subjected to temperature increase, and during a process of temperature change, the secondary amplification double-stranded product formed with the probe (P) will melt at a certain temperature, so as to cause a change in the distance between the detection groups, thereby generating a signal that may be detected by the instrument. The signals detected by the instrument are shown as a melting curve with the change of the temperature, and when a Tm value at a peak position of the melting curve is within a range of a characteristic Tm value of the target in the sample to be detected in a specific detection channel, it indicates that there is a target contained in the sample to be detected. The specific detection channel is a fluorescent channel corresponding to the first detection group on the probe (P), and the characteristic Tm value is a melting temperature of the secondary amplification double-stranded product formed with the probe (P) (a melting temperature of the secondary amplification double-stranded product formed with the probe (P)).

In some other preferred embodiments of the present invention, the primer probe set comprises:
one first primer, sequentially comprising a probe anchoring region (A) and a target sequence binding region 1 from 5' end to 3' end; wherein, the target sequence binding region 1 is a sequence that can specifically bind with a target sequence in a paired manner, and the probe anchoring region (A) is a freely designed sequence that does not bind with any target sequence in a paired manner, and has a sequence complementary with 6-35 bases of a primer anchoring region (A') of a probe (P); and there may be 0-20 bases spaced between the probe anchoring region (A) and the target sequence binding region 1;
one probe (P), being a freely designed sequence that does not bind with any target sequence in a paired manner, and sequentially comprising a primer anchoring region (A') and a probe signal detection region (H) from 5' end to 3' end; wherein a sequence of the primer anchoring region (A') is complementary with a sequence of the probe anchoring region (A) of the first primer; and a second detection group is labeled at a base position 3-140 angstroms apart from a first detection group, wherein the first detection group may be a fluorescence reporter group, and the second detection group may be a quencher group or other labeling groups that can produce a signal change with the first detection group via fluorescence resonance energy transfer (FRET); and
one second primer, sequentially comprising an extension blocking region (M), a primer signal detection region (h) and a target sequence binding region 2 from 5' end to 3' end, wherein the target sequence binding region 2 is a sequence that can specifically bind with the target sequence in a paired manner; the primer signal detection region (h) in the second primer is a freely designed sequence that does not bind with any target sequence in a paired manner, and has a sequence partially or completely identical to that of the probe signal detection region (H) of the probe (P); and there may be 0-20 bases spaced between the primer signal detection region (h) and the target sequence binding region 2.

The pre-amplification product contains one single-stranded pre-amplification product sequentially comprising the probe anchoring region (A), the target sequence, a reverse-complementary sequence (h') of the primer signal detection region (h), and a reverse-complementary sequence (M') of the extension blocking region (M) from 5' end to 3' end. The reverse-complementary sequence (M') of the extention blocking region (M) is a sequence that is not complementary with any part of the probe (P).

In some other preferred embodiments of the present invention, the primer probe set comprises:
one first primer, sequentially comprising a probe anchoring region (A) and a target sequence binding region 1 from 5' end to 3' end; wherein, the target sequence binding region 1 is a sequence that can specifically bind with a target sequence in a paired manner, and the probe anchoring region (A) is a freely designed sequence that does not bind with any target sequence in a paired manner, and has a sequence complementary with 6-35 bases of the primer anchoring region (A') of the probe (P); and there may be 0-20 bases spaced between the probe anchoring region (A) and the target sequence binding region 1;
one probe (P), being a freely designed sequence that does not bind with any target sequence in a paired manner, and sequentially comprising a primer anchoring region (A') and a probe signal detection region (H) from 5' end to 3' end; wherein a sequence of the primer anchoring region (A') is complementary with a sequence of the probe anchoring region (A) of the first primer; and a second detection group is labeled at a base position 3-140 angstroms apart from a first detection group, wherein the first detection group may be a fluorescence reporter group, and the second detection group may be a quencher group or other labeling groups that can produce a signal change with the first detection group via fluorescence resonance energy transfer (FRET); and
one second primer, sequentially comprising an extension blocking region (M), a primer signal detection region (h) and a target sequence binding region 2 from 5' end to 3' end, wherein the target sequence binding region 2 is a sequence that can specifically bind with the target sequence in a paired manner; all of the primer signal detection regions (h) in different second primers are a freely designed sequence that does not bind with any target sequence in a paired manner, and has a sequence partially or completely identical to that of the probe signal detection region (H) of the probe (P); and there may be 0-20 bases spaced between the primer signal detection region (h) and the target sequence binding region 2.

The pre-amplification product contains one single-stranded pre-amplification product sequentially comprising the probe anchoring region (A), the target sequence, a reverse-complementary sequence (h') of the primer signal detection region, and a reverse-complementary sequence (M') of the extension blocking region (M) from 5' end to 3' end. The reverse-complementary sequence (M') of the extention blocking region (M) is a sequence that is not complementary with any part of the probe (P).

In the present invention, a principle of performing PCR detection to a sample to be detected by using the above primer probe is as follows:
During PCR amplification, the first primer and second primer in the primer probe specifically bind with the target sequence respectively and are subjected to amplification to produce a double-stranded pre-amplification product, wherein one single-stranded pre-amplification product (S1) sequentially comprises the probe anchoring region (A), the target sequence, the reverse-complementary sequence (h') of the primer signal detection region (h), and the reverse-complementary sequence (M') of the extension blocking region (M) from 5' end to 3' end.

After the double-stranded pre-amplification product is produced, the probe signal detection region (H) of the probe (P) is complementarily paired with the reverse-complementary sequence (h') of the primer signal detection region at the 3' end of the single-stranded pre-amplification product (S1), and the primer anchoring region (A') of the probe (P) is complementarily paired with the probe anchoring region (A) at 5' end of the single-stranded pre-amplification product (S1), and the reverse-complementary sequence (h') in the single-stranded pre-amplification product (S1) specifically binds with probe (P) and extends by 0 base to form a double-stranded product, so as to obtain a partially or completely reverse-complementary sequence of probe (P), that is, a hybridization in which the single-stranded pre-amplification product (S1) is used as a primer and the probe (P) is used as a template is completed, to obtain a hybridization double-stranded product formed with the probe (P). At this time, a partially or completely double-stranded state is formed by the probe (P) and the single-stranded pre-amplification product (S1), and a distance between the first detection group and the second detection group is changed, thereby generating a signal that may be detected by an instrument.

After the PCR amplification is completed, the resultant is subjected to temperature increase, and during a process of temperature change, the hybridization double-stranded product formed with the probe (P) will melt at a certain temperature, so as to cause a change in the distance between the detection groups, thereby generating a signal that may be detected by the instrument. The signals detected by the instrument are shown as a melting curve with the change of the temperature, and when a Tm value at a peak position of the melting curve is within a range of a characteristic Tm value of the target in the sample to be detected in a specific detection channel, it indicates that there is a target contained in the sample to be detected. The specific detection channel is a fluorescent channel corresponding to the first detection group on the probe (P), and the characteristic Tm value is a melting temperature of the hybridization double-stranded product formed with the probe (P) (a melting temperature of the hybridization double-stranded product formed with the probe (P)).

In a second aspect, the present invention provides a kit for PCR detection, comprising the primer probe for PCR detection of the first aspect; preferably further comprising an amplification reagent; further preferably, the amplification reagent comprises a DNA polymerase and dNTPs; and still further preferably, when a nucleic acid template is RNA, the amplification reagent further comprises a reverse transcriptase.

In the present invention, the amplification reagent may further comprise some reagents that promote PCR reactions, such as KCl, MgCl₂, Tris-HCl, dithiothreitol (DTT), (NH₄)₂SO₄, etc.

In a third aspect, the present invention provides a primer probe set for PCR detection, characterized by comprising a probe (P), at least one first primer, and at least two second primers;
the first primer comprises a target sequence binding region 1, and different target sequence binding regions 1 are sequences that can specifically bind with different target sequences in a paired manner;
the probe (P) is a sequence that does not bind with any target sequence in a paired manner, and comprises a probe signal detection region (H); and the probe (P) is labeled with a detection label; and
the at least two second primers are different from each other, each independently comprising a primer signal detection region (h) and a target sequence binding region 2; wherein, the target sequence binding regions 2 in different second primers are sequences that specifically bind with different target sequences in a paired manner, all of the primer signal detection regions (h) in different second primers are sequences that do not bind with any target sequence in a paired manner, and a complementary sequence (h') of the primer signal detection region (h) specifically binds with the probe signal detection region (H) of the probe (P) in a paired manner; the target sequence binding region 2 is located at 3' end of the primer signal detection region (h); and the primer signal detection regions (h) of different second primers are different from each other in sequence.

In the present invention, the first primer and the second primer specifically bind with the target respectively to produce a pre-amplification product, and the pre-amplification product contains one single-stranded pre-amplification product having the complementary sequence (h') of the primer signal detection region (h), the complementary sequence (h') in the single-stranded pre-amplification product specifically binds with the probe (P) and extends by ≥ 0 bases to form a double-stranded product, and the formation of the double-stranded product causes the probe to produce a signal change.

In the present invention, the single-stranded pre-amplification products formed by different second primers are different, and the double-stranded products formed by combining different single-stranded pre-amplification products with the probe (P) are different, and the Tm values of different double-stranded products are different.

The expression "the Tm values are different" means that the melting temperatures are different, that is, different double-stranded products may be separated from each other by means of melting curves.

In the present invention, the first primer is a forward primer (F), and the second primer is a reverse primer (R); or
the first primer is a reverse primer (R), and the second primer is a forward primer (F).

In some embodiments of the present invention, the first primer is a forward primer (F), and the second primer is a reverse primer (R).

In the present invention, the complementary sequence (h') of the primer signal detection region (h) is a forward-complementary sequence (h') of the primer signal detection region (h) or a reverse-complementary sequence (h') of the primer signal detection region (h).

In the present invention, the complementary sequence (h') of the primer signal detection region (h) binds with the probe signal detection region (H) of the probe (P) in a forward-complementary paired manner or in a reverse-complementary paired manner.

In some embodiments of the present invention, the complementary sequence (h') of the primer signal detection region (h) is the reverse-complementary sequence (h') of the primer signal detection region (h).

In some embodiments of the present invention, the complementary sequence (h') of the primer signal detection region (h) binds with the probe signal detection region (H) of the probe (P) in a reverse-complementary paired manner.

In some embodiments of the present invention, the complementary sequence (h') of the primer signal detection region (h) is the reverse-complementary sequence (h') of the primer signal detection region (h); and the complementary sequence (h') of the primer signal detection region (h) binds with the probe signal detection region (H) of the probe (P) in a reverse-complementary paired manner.

In some embodiments of the present invention, the double-stranded product is a secondary amplification double-stranded product.

In the present invention, "different detection labels" mean achieving detection under different detection channels. The detection label comprises a first detection group and a second detection group, and the first detection group and the second detection group generate a signal change via a distance change; and one of the first detection group and the second detection group may be a fluorescence group, and the other may be a quencher group or other groups that can produce a signal change with a fluorescence group via fluorescence resonance energy transfer.

The fluorescence groups of different probes are different, but the quencher groups thereof may be the same.

In some embodiments of the present invention, the primer probe set for PCR detection comprises 1-8 probes (P), 1-88 first primers, and 2-88 second primers.

In some embodiments of the present invention, sequences of primer signal detection regions (h) of different reverse primers (R) are different from each other.

In some specific embodiments of the present invention, the first primer is a forward primer (F), and the second primer is a reverse primer (R).

In some specific embodiments of the present invention, the double-stranded product is a secondary amplification product.

In some specific embodiments of the present invention, the primer probe set comprises at least one probe (P), at least one forward primer (F), and at least two reverse primers (R); and preferably, the primer probe set comprises at least one probe (P), at least two forward primers (F), and at least two reverse primers (R); wherein,
the different forward primers (F) each independently contains target sequence binding regions 1 that specifically bind with different target sequences in a paired manner;
the probes (P) is a sequence that does not bind with any target sequence in a paired manner, and comprises a probe signal detection region (H); and sequences of the probe signal detection regions (H) of different probes (P) are different, and the labeled detection groups thereof are different;
different reverse primers (R) each sequentially comprise a primer signal detection region (h) and a target sequence binding region 2 from 5' end to 3' end; wherein, the target sequence binding regions 2 of different reverse primers (R) are sequences that specifically bind with different target sequences in a paired manner, and each of the primer signal detection regions (h) of different reverse primers (R) is a sequence that does not bind with any target sequence in a paired manner, but has a sequence partially or completely identical to that of the probe signal detection region (H) of probe (P);
the forward primer (F) and the reverse primer (R) specifically bind with the target respectively to produce a pre-amplification product, and the pre-amplification product contains one single-stranded pre-amplification product having a reverse complementary sequence (h') of the primer signal detection region (h), the reverse complementary sequence (h') in the single-stranded pre-amplification product specifically binds with the probe (P) and extends to form a secondary amplicaiton double-stranded product, and the formation of the secondary amplicaiton double-stranded product causes the probe to produce a signal change; and
the single-stranded pre-amplification products formed by different forward primers (F) and reverse primers (R) are different, and the Tm values of different secondary amplicaiton double-stranded products formed by the single-stranded pre-amplification products are different.

In some preferred embodiments of the present invention, sequences of the primer signal detection regions (h) of different reverse primers (R) are different from each other.

In the present invention, the expression "sequences of the primer signal detection regions (h) of different reverse primers (R) are different from each other" means that bases and/or lengths of the sequences of the primer signal detection regions (h) of different reverse primers (R) are different, such that different single-stranded pre-amplification products containing the reverse-complementary sequences (h') of different primer signal detection regions (h) combine with the probe and extend to form different secondary amplification double-stranded products. The melting temperature curves of different secondary amplification double-stranded products may be separated from each other.

Due to the fact that the melting temperature of the secondary amplification double-stranded product formed with the probe (P) is detected during melting curve analysis, it is possible to adjust the melting temperature of the secondary amplification double-stranded product formed with the probe (P) by adjusting the base and length of the sequence of the primer signal detection region (h) of the reverse primer (R) to obtain the reverse-complementary sequence (h') of the primer signal detection region of the single-stranded pre-amplification product (S1) that is partially or completely reverse complementary with the probe signal detection region (H) of the probe (P). For example, it is possible to increase or decrease the melting temperature of the secondary amplification double-stranded product formed with the probe (P) by adjusting the length or the base of the sequence of the primer signal detection region (h), or adjusting a position at which the reverse-complementary sequence (h') of the primer signal detection is reversely complementary with the probe signal detection region (H) of the probe (P).

In the present invention, in the primer probe set, there are a plurarity of forward primers (F) and reverse primers (R) targeting different target sequences. When the above primer probe set is used for multiplex PCR detection, different target sequences may be identified by means of fluorescence channels or melting temperatures; and when different target sequences are identified by means of fluorescence channels, different probes (P) may be used for different target sequences, and when different target sequences are identified by means of melting temperatures, a same probe (P) may be shared by different target sequences.

In some embodiments of the present invention, the at least one forward primer (F) each sequentially comprise a probe anchoring region (A) and a target sequence binding region 1 from 5' end to 3' end; wherein, the probe anchoring region (A) is a sequence that does not bind with any target sequence in a paired manner; and the target sequence binding regions 1 in different forward primers (F) are different in sequence;
the probe (P) is a sequence that does not bind with any target sequence in a paired manner, and comprises a primer anchoring region (A') and a probe signal detection region (H); and the primer anchoring region (A') is a sequence complementary with that of the probe anchoring region (A) of the forward primer (F).

In some embodiments of the present invention, at least one of the first primers further comprises a probe anchoring region (A); wherein the probe anchoring region (A) is a sequence that does not bind with any target sequence in a paired manner, and is located at 5' end of the target sequence binding region 1; and
the probe (P) further comprises a primer anchoring region (A'); and the primer anchoring region (A') is a sequence complementary with that of the probe anchoring region (A) of the first primer. In some embodiments of the present invention, at least one of the first primers sequentially comprise a probe anchoring region (A) and a target sequence binding region 1 from 5' end to 3' end; wherein, the probe anchoring region (A) is a sequence that does not bind with any target sequence in a paired manner; and the target sequence binding regions 1 in different first primers are different in sequence.

In some preferred embodiments of the present invention, the primer anchoring regions (A') and/or probe signal detection regions (H) of different probes (P) are different in sequence, and the labeled detection groups thereof are different.

In the present invention, the probe anchoring regions (A) of different forward primers may be the same or may be different. When the probe anchoring regions (A) of different forward primers are different, by adjusting the sequences and/or lengths of the probe anchoring regions A of different forward primers and the primer signal detection regions of different reverse primers, such that different single-stranded pre-amplification products contain different probe anchoring regions (A) and reverse-complementary sequences (h') of the primer signal detection regions (h), and different secondary amplification double-stranded products are formed by combining different single-stranded pre-amplification products with the probe and extending. The melting temperature curves of different secondary amplification double-stranded products may be differentiated from each other.

Due to the fact that the melting temperature of the secondary amplification double-stranded product formed with the probe (P) is detected during melting curve analysis, it is possible to adjust the melting temperature of the secondary amplification double-stranded product formed with the probe (P) by adjusting the base and length of the sequence of the probe anchoring region (A) of the forward primer (F) and/or the primer signal detection region (h) of the reverse primer (R). For example, it is possible to increase or decrease the melting temperature of the secondary amplification double-stranded product formed with the probe (P) by adjusting the length or the base of the sequence of the probe anchoring region (A), or adjusting the length or the base of the sequence of the primer signal detection region (h), or adjusting a position at which the probe anchoring region and the primer anchoring region (A') of the probe (P) is complementary with eath other, or adjusting a position at which the reverse-complementary sequence (h') of the primer signal detection region is reversely complementary with the probe signal detection region (H) of the probe (P).

In some embodiments of the present invention, there are 0-20 bases independently spaced between the primer signal detection region (h) and the target sequence binding region 2 of the second primer. In some embodiments of the present invention, there are 0-20 bases independently spaced between the primer signal detection regions (h) and the target sequence binding regions 2 of different reverse primers (R).

In some embodiments of the present invention, the detection label is a detection group.

In some embodiments of the present invention, the detection label comprises a first detection group and a second detection group, and the first detection group and the second detection group generate a signal change via a distance change; preferably, the first detection group is spaced from the second detection group by 3-250 angstroms; preferably, by 3-201 angstroms; more preferably, by 3-140 angstroms; and further preferably, the first detection group is a fluorescence reporter group, and the second detection group is a quencher group or other labeling groups that can produce a signal change with the first detection group via fluorescence resonance energy transfer.

In some embodiments of the present invention, the probe (P) comprises 1-2 primer anchoring regions (A') and 1-2 probe signal detection regions (H).

In some embodiments of the present invention, the probe (P) is any one structure of 1) to 4);
1) the probe (P) comprising one primer anchoring region (A') and one probe signal detection region (H);
2) the probe (P) compring one probe signal detection region (H) and two primer anchoring regions (A'), wherein the two primer anchoring regions (A') are located on two sides of the probe signal detection region (H), respectively, and the two primer anchoring regions (A') are different in sequence;
3) the probe (P) comprising one primer anchoring region (A') and two probe signal detection regions (H), wherein the two probe signal detection regions (H) are located on two sides of the primer anchoring region (A'), and the two probe signal detection regions (H) are different in sequence; and
4) the probe (P) comprising two primer anchoring regions (A') and two probe signal detection regions (H), wherein the primer anchoring regions (A') and the probe signal detection regions (H) are arranged at intervals, the two primer anchoring regions (A') are different in sequence, and the two probe signal detection regions (H) are different in sequence.

In some embodiments of the present invention, a blocking region is contained at 3' end of the probe (P).

In some embodiments of the present invention, an extention blocking region (M) is contained at 5' end of at least one of the second primer.

In some embodiments of the present invention, the pre-amplification product contains one single-stranded pre-amplification product having a complementary sequence (h') of the primer signal detection region (h) and a complementary sequence (M') of the extension blocking region (M); and the complementary sequence (M') of the extention blocking region (M) is a sequence that does not specifically bind with any part of the probe (P) in a paired manner.

In the present invention, the complementary sequence (M') of the extention blocking region (M) is a forward-complementary sequence (M') of the extention blocking region (M) or a reverse-complementary sequence (M') of the extention blocking region (M); and/or
the complementary sequence (M') of the extention blocking region (M) is a sequence that does not bind with any part of the probe (P) in a forward-complementary paired manner or in a reverse-complementary paired manner.

In some embodiments of the present invention, the complementary sequence (M') of the extention blocking region (M) is the reverse-complementary sequence (M') of the extention blocking region (M).

In some specific embodiments of the present invention, the first primer sequentially comprises a probe anchoring region (A) and a target sequence binding region 1 from 5' end to 3' end; wherein, the probe anchoring region (A) is a sequence that does not bind with any target sequence in a paired manner; and there may or may not be a base spacing between the probe anchoring region (A) and the target sequence binding region 1;
the probe (P) is a sequence that does not bind with any target sequence in a paired manner, and comprises a primer anchoring region (A') and a probe signal detection region (H); the primer anchoring region (A') is a sequence complementary with the probe anchoring region (A) of the first primer; and there may or may not be a base spacing between the primer anchoring region (A') and the probe signal detection region (H);
at least one of the second primers sequentially comprises an extension blocking region (M), a primer signal detection region (h), and a target sequence binding region 2 from 5' end to 3' end; and there may or may not be a base spacing between the extention blocking region (M) and the primer signal detection region (h), as well as between the primer signal detection region (h) and the target sequence binding region 2; and
the pre-amplification product contains a single-stranded pre-amplification product comprising the probe anchoring region (A), the target sequence, a reverse-complementary sequence (h') of the primer signal detection region (h), and a reverse-complementary sequence (M') of the extension blocking region (M) from 5' end to 3' end.

In the present invention, when there is no extention blocking region (M) contained at 5'end of the second primer, the reverse-complementary sequence (h') in the obtained single-stranded pre-amplification product specifically binds with the probe (P) and extends by ≥ 0 bases to form a double-stranded product.

Specifically, if the reverse-complementary sequence (h') in the single-stranded pre-amplification product does not specifically bind with the probe (P) at 5' end of the probe (P), it specifically binds and extends to the 5' end of the probe (P), i.e. it extends by >0 base, to form a double-stranded product; and the double-stranded product is a secondary amplication double-stranded product.

If there is no extention blocking region (M) contained at 5'end of the second primer, and the reverse-complementary sequence (h') in the obtained single-stranded pre-amplification product specifically binds with the probe (P) at the 5'end of the probe (P), it specifically binds and extends by 0 base to form a double-stranded product. That is, the double-stranded product is formed by hybridization after specifical binding, and the double-stranded product is a hybridization double-stranded product.

In the present invention, when the extention blocking region (M) is contained at 5'end of the second primer, due to the action of the extension blocking region (M), the reverse-complementary sequence (h') in the obtained single-stranded pre-amplification product specifically binds with the probe (P) and extends by 0 base to form a double-stranded product. That is, the double-stranded product is formed by hybridization after specifical binding, and the double-stranded product is a hybridization double-stranded product.

In the cases of extending by >0 base and extending by 0 base, the formation of both of the secondary amplification double-stranded product and the hybridization double-stranded product cause a probe signal change, thereby generating a detectable signal change.

In some embodiments of the present invention, a Tm value of the primer signal detection region (h) is simultaneously lower than a Tm value of the target sequence binding region 1 in the first primer and a Tm value of the target sequence binding region 2 in the second primer.

In some embodiments of the present invention, the Tm value of the primer signal detection region (h) is simultaneously lower than a Tm value of the target sequence binding region 1 in the forward primer (F) and a Tm value of the target sequence binding region 2 in the reverse primer (R).

In some embodiments of the present invention, a Tm value of the primer anchoring region (A') is higher than the Tm value of the primer signal detection region (h).

In the present invention, by limiting the Tm value of the primer signal detection region (h) and the Tm value of the primer anchoring region (A') within the aforementioned range, the generation of primer dimers during amplification may be effectively avoided.

In some embodiments of the present invention, there are 0-20 bases spaced between the probe anchoring region (A) and the target sequence binding region 1 in the first primer.

In some embodiments of the present invention, there are 0-20 bases independently spaced between the probe anchoring regions (A) and the target sequence binding region s 1 of different forward primers (F).

In some embodiments of the present invention, different forward primers (F) and different reverse primers (R) each independently have a length of 15-80 bases; preferably, the target sequence binding region 1 and the target sequence binding region 2 each independently have a Tm value of 40°C-80°C, and a GC content of 20%-80%; and the primer signal detection region (h) has a length of 5-65 bases, a Tm value of 30-80°C, and a GC content of 20-80%.

In some other embodiments of the present invention, the probe (P) has a length of 20-100 bases; and preferably, the probe signal detection region (H) has a length of 5-65 bases, a Tm value of 30-85°C, and a GC content of 20-80%.

In some other embodiments of the present invention, the probe anchoring region (A) has a length of 6-35 bases, a Tm value of 40°C-80°C, and a GC content of 30%-80%.

In some embodiments of the present invention, the primer anchoring region (A') has a length of 6-35 bases, a Tm value of 40°C-85°C, and a GC content of 20%-80%; and preferably, a Tm value of the primer anchoring region (A') is higher than a Tm value of the primer signal detection region (h).

In the present invention, the Tm value of the primer anchoring region (A') is allowed to be higher than the Tm value of the primer signal detection region (h), such that the binding of a reverse-complementary sequence (h') of the primer signal detection region to the probe needs the help of the probe anchoring region (A').

In a fourth aspect, the present invention provides a kit for PCR detection, comprising the primer probe set for PCR detection as described in the third aspect of the present invention; preferably, it further comprises an amplification reagent; further preferably, the amplification reagent comprises a DNA polymerase and dNTPs; and still further preferably, when a nucleic acid template is RNA, the amplification reagent further comprises a reverse transcriptase.

In the present invention, the amplification reagent may further comprise some reagents that promote PCR reactions, such as KCl, MgCl₂, Tris-HCl, dithiothreitol (DTT), (NH₄)₂SO₄, etc.

In a fifth aspect, the present invention provides a primer probe composition for PCR detection, comprising at least one primer probe set as described in the third aspect.

In some embodiments of the present invention, the primer probe composition comprises 1-8 primer probe sets as described in a third aspect.

In some embodiments of the present invention, the at least two different primer probe sets are different in probe (P), the probes (P) of different primer probe sets are different in sequence, and preferably, the probes (P) of different primer probe sets are different in sequence and detection label.

In some embodiments of the present invention, 2-8 different primer probe sets are different in probe (P).

In a sixth aspect, the present invention provides a use of the primer probe composition for PCR detection as described in the fifth aspect in preparation of reagents for detecting various different targets.

In a seventh aspect, the present invention provides a kit for PCR detection, comprising the primer probe composition for PCR detection as described in the fifth aspect, preferably it further comprises an amplification reagent; further preferably, the amplification reagent comprises a DNA polymerase and dNTPs; and still further preferably, when a nucleic acid template is RNA, the amplification reagent further comprises a reverse transcriptase.

In an eighth aspect, the present invention provides a method for performing PCR detection to a sample to be detected by using the kit as described in any one of the second aspect, the fourth aspect and the seventh aspect, comprising the following steps:
S1, mixing the first primer, the second primer, and the probe (P) with the amplification reagent and the sample to be detected, to obtain a reaction system;
S2, performing PCR amplification to the reaction system; and
S3, analyzing whether there is a target.

The method for performing PCR detection described in the present invention may be used for diagnostic and therapeutic purposes, and may also be used for non-diagnostic and non-therapeutic purposes.

In some embodiments of the present invention, the step S3 comprises the following steps:
S3-1, obtaining a signal change generated by the PCR amplification in step S2, and judging whether there is a target in the sample to be detected based on the signal change; and/or
S3-2, heating a product obtained by the PCR amplification in step S2, and judging whether there is a target contained in the sample to be detected based on a signal change.

The above PCR detection method is a fluorescent PCR detection method.

In some embodiments of the present invention, in the reaction system obtained in step S1, a concentration of the first primer is 30 nM-1000 nM, a concentration of the second primer is 30 nM-500nM, and a concentration of the probe (P) is 150 nM-1200 nM.

In some embodiments of the present invention, the nucleic acid template is from a serum sample, a plasma sample, a whole blood sample, a sputum sample, a swab sample, a lavage fluid sample, a fresh tissue sample, a formalin fixed paraffin embedded tissue (FFPE).

In some embodiments of the present invention, the method for performing PCR detection to a sample to be detected by using the kit comprises the following steps:
S1, mixing the forward primer (F), the reverse primer (R), and the probe (P) with the amplification reagent and the sample to be detected, to obtain a reaction system;
S2, performing PCR amplification to the reaction system; and
S3, analyzing whether there is a target.

In some embodiments of the present invention, step S3 comprises the following steps:
S3-1, obtaining a signal change generated by the PCR amplification in step S2, and judging whether there is a target in the sample to be detected based on the signal change; and/or
S3-2, heating a product obtained by the PCR amplification in step S2, and judging whether there is a target contained in the sample to be detected based on a signal change.

In some embodiments of the present invention, the step S3 comprises the following steps:
S3-1, obtaining a signal change generated by the PCR amplification in step S2, and judging whether there is a target in the sample to be detected based on the signal change; and/or
S3-2, performing melting curve analysis to a product obtained by the PCR amplification in step S2, and judging whether there is a target contained in the sample to be detected.

In some embodiments of the present invention, the step S2 comprises the following steps:
S2-1, randomly allocating the reaction system into 500 or more reaction units, wherein each reaction unit contains one target of the sample to be detected or does not contain a target of the sample to be detected; and/or,
S2-2, performing PCR amplification to all of the reaction units;
and/or, the step S3 comprises the following steps:
S3, obtaining a signal situation of each reaction unit after the PCR amplification in step S2, and judging whether there is a target based on presence or absence of a signal.

The above PCR detection method is a digital PCR detection method.

In some embodiments of the present invention, at S2-1, the reaction system is randomly allocated to 500-20000 reaction units, wherein each reaction unit contains one target of the sample to be detected or does not contain a target of the sample to be detected.

In some other embodiments of the present invention, in the reaction system obtained in step S1, a concentration of the forward primer (F) is 30 nM-1000 nM, a concentration of the reverse primer (R) is 30 nM-1000 nM, and a concentration of the probe (P) is 100 nM-1200 nM.

In some embodiments of the present invention, in step S2, when the nucleic acid template is DNA, a PCR amplification program is as follows:
performing pre-denaturation at 90°C-96°C for 3-15 minutes; performing denaturation at 90°C-95°C for 10-60 seconds, and performing annealing and extension at 50°C-75°C for 30-90 seconds, for 35-50 cycles; an performing denaturation at 90°C-95°C for 10-600 seconds, and performing annealing and extension at 30°C-55°C for 10-600 seconds, for 1-20 cycles.

In some embodiments of the present invention, in step S2, when the nucleic acid template is DNA, a PCR amplification program is as follows:
performing pre-denaturation at about 85°C-about 105°C for about 0-about 15 minutes; performing denaturation at about 85°C-about 105°C for about 1-about 60 seconds, and performing annealing and elongation at about 40°C-about 75°C for about 3-about 90 seconds, for about 20-about 60 cycles; performing denaturation at about 85°C-about 105°C for about 1-about 60 seconds, and performing annealing and elongation at about 20°C-about 75°C for about 3-about 90 seconds, for 1-20 cycles.

In some specific embodiments of the present invention, in step S2, when the nucleic acid template is DNA, the PCR amplification program is as follows: performing pre-denaturation at 95°C for 3 minutes; performing denaturation at 94°C for 10 seconds, and performing annealing and extension at 56°C for 30 seconds, for a total of 45 cycles; and performing denaturation at 94°C for 150 seconds, and performing annealing and extension at 40°C for 300 seconds, for 1 cycle.

In some embodiments of the present invention, in step S2, when the nucleic acid template is RNA, the PCR amplification program is as follows:
performing reverse transcription at 50-60°C for 5-15 minutes; performing pre-denaturation at 90°C-96°C for 3-15 minutes; performing denaturation at 90°C-95°C for 10-60 seconds, and performing annealing and extension at 50°C-75°C for 30-90 seconds, for 35-50 cycles; and performing denaturation at 90°C-95°C for 10-600 seconds, and performing annealing and extension at 30°C-55°C for 10-600 seconds, for 1-20 cycles.

In some embodiments of the present invention, in step S2, when the nucleic acid template is RNA, the PCR amplification program is as follows:
performing reverse transcription at about 30-about 65°C for about 2-about 30 minutes; performing pre-denaturation at about 85°C-about 105°C for about 0-about 15 minutes; performing denaturation at about 85°C-about 105°C for about 1-about 60 seconds, and performing annealing and elongation at about 40°C-about 75°C for about 3-about 90 seconds, for 20-60 cycles; and
performing denaturation at about 85°C-about 105°C for about 1-about 60 seconds, and performing annealing and elongation at about 20°C-about 75°C for about 3-about 90 seconds, for 1-20 cycles.

In some specific embodiments of the present invention, in step S2, when the nucleic acid template is RNA, the PCR amplification program is as follows: performing reverse transcription at 55°C for 15 minutes; performing pre-denaturation at 95°C for 3 minutes; performing denaturation at 94°C for 10 seconds, and performingannealing and extension at 56°C for 30 seconds, for a total of 45 cycles; and performing denaturation at 94°C for 150 seconds, and performing annealing and extension at 40°C for 300 seconds, for 1 cycle.

In a ninth aspect, the present invention provides a method for performing PCR detection to a sample to be detected by using the kit as described in any one of the second aspect, the fourth aspect and the seventh aspect, comprising the following steps:
allowing the first primer and the second primer to specifically bind with a target respectively, to produce, after amplification, a pre-amplification product containing a complementary sequence (h') of the primer signal detection region (h);
allowing the complementary sequence (h') of the primer signal detection region (h) to specifically bind with the probe (P) and extend to form a secondary amplification double-stranded product; the formation of the double-stranded product causes the probe to produce a detectable signal change, and judging whether there is a target in the sample to be detected based on the signal change; and/or
heating the double-stranded product, wherein a melting of the double-stranded product causes the probe to produce a detectable signal change, and judging whether there is a target contained in the sample to be detected based on the signal change.

In the present invention, heating the double-stranded product means increasing a temperature of the double-stranded product to a value higher than a Tm value of the double-stranded product.

In some embodiments of the present invention, a heating rate is 0.05°C/s, a fluorescence signal is collected under a condition of 35-95°C, and whether there is a target contained in the sample to be detected is determined based on a signal change.

In some specific embodiments of the present invention, a heating rate is 0.05°C/s, a fluorescence signal is collected under a condition of 40-90°C, and whether there is a target contained in the sample to be detected is determined based on a signal change; and preferably, the heating rate is 0.05°C/s, the temperature is gradually increased from 40°C to 90°C, a fluorescence signal is real-time collected during temperature increase, a melting curve is obtained after the temperature increase is completed, and whether there is a target contained in the sample to be detected is determined based on a signal change.

In some specific embodiments of the present invention, a heating rate is 0.05°C/s, a fluorescence signal is collected under a condition of 56-90°C, and whether there is a target contained in the sample to be detected is determined based on a signal change; and preferably, the heating rate is 0.05°C/s, the temperature is gradually increased from 56°C to 90°C, a fluorescence signal is real-time collected during temperature increase, a melting curve is obtained after the temperature increase is completed, and whether there is a target contained in the sample to be detected is determined based on a signal change.

Preferably, the signal change refers to a fluorescence signal change. For example, when a temperature at which a maximal signal change occurs is within the range of a Tm value of the double-stranded product formed by the probe (P) of the target in the sample to be detected in a specific detection channel, it indicates that there is a target contained in the sample to be detected. The specific detection channel is a fluorescent channel corresponding to the detection group on the probe (P).

The temperature at which a maximal signal change occurs is a Tm value at a peak position.

In some embodiments of the present invention, when a Tm value at a peak position of the obtained melting curve is within a range of a Tm value of the double-stranded product formed by the target in the sample to be detected with the probe (P) in a specific detection channel, it indicates that there is a target contained in the sample to be detected; and preferably, the specific detection channel is a fluorescent channel corresponding to the first detection group on the probe (P).

In some embodiments of the present invention, when there is no target to be detected, the primer anchoring region (A') of the probe (P) is complementary with the probe anchoring region (A) in the first primer, and the other part of the probe (P) is in a single-stranded state or in other secondary structures. At this time, a distance between the first detection group and the second detection group is relatively close, and the efficiency of fluorescence resonance energy transfer is relatively high.

When a target to be detected is present, the first primer and the second primer specifically bind with a target sequence respectively and extend to produce a double-stranded amplification product, and then a secondary amplification is performed by taking one single-stranded amplification product (S1) of the double-stranded amplification product as a primer and taking the probe (P) as a template, to obtain a double-stranded product formed with the probe (P). At this time, the distance between the first detection group and the second detection group is increased, and the efficiency of fluorescence resonance energy transfer is decreased, such that a fluorescence signal change is produced and may be detected by an instrument. After the completion of PCR amplification, when a melting curve analysis is performed, the double-stranded product formed with the probe (P) will melt, at this time, the distance between the first detection group and the second detection group is decreased, and the efficiency of fluorescence resonance energy transfer is increased, such that a fluorescence signal change is produced again, which may be used for melting curve analysis. When a Tm value at a peak position of the melting curve is detected to be within a range of a characteristic Tm value of the target in the sample to be detected in a specific detection channel, it indicates that there is a target contained in the sample to be detected. The specific detection channel is a fluorescent channel corresponding to the first detection group on the probe (P), and the characteristic Tm value is a melting temperature of the double-stranded product formed with the probe (P).

In some specific embodiments of the present invention, the method comrprises the following steps: allowing the forward primer (F) and the reverse primer (R) to specifically bind with the target respectively, to produce, after amplification, a pre-amplification product containing a reverse complementary sequence (h') of the primer signal detection region (h);
allowing the reverse complementary sequence (h') of the primer signal detection region (h) to specifically bind with the probe (P) and extend to form a secondary amplification double-stranded product;
the formation of the double-stranded product causes the probe to produce a detectable signal change, and judging whether there is a target in the sample to be detected based on the signal change; and/or
performing melting curve analysis to the secondary amplification double-stranded product and judging whether there is a target contained in the sample to be detected.

In some embodiments of the present invention, a specific operation of the melting curve analysis is as follows: gradually increasing the temperature from 35-45°C to 85-95°C, and real-time collecting a fluorescence signal during temperature increase, to obtain a melting curve after the temperature increase is completed; preferably, a heating rate is 0.03-0.08°C/s.

In some specific embodiments of the present invention, a specific operation of the melting curve analysis is as follows: gradually increasing the temperature from 40°C to 85-87°C, and real-time collecting a fluorescence signal during temperature increas, to obtain a melting curve after the temperature increase is completed; wherein, a heating rate is 0.05°C/s.

In some embodiments of the present invention, when a Tm value at a peak position of the obtained melting curve is within a range of a characteristic Tm value of the target in the sample to be detected in a specific detection channel, it indicates that there is a target contained in the sample to be detected; and preferably, the specific detection channel is a fluorescent channel corresponding to the first detection group on the probe (P), and the characteristic Tm value is a melting temperature of the secondary amplification double-stranded product formed with the probe (P).

When there is no target to be detected, the primer anchoring region (A') of the probe (P) is complementary with the probe anchoring region (A) of the forward primer (F), and the other part of the probe (P) is in a single-stranded state, and a curly shape is formed due to molecular flexibility. At this time, a distance between the first detection group and the second detection group is relatively close, and the efficiency of fluorescence resonance energy transfer is relatively high. When a target to be detected is present, the forward primer (F) and the reverse primer (R) specifically bind with a target sequence respectively and extend to produce a double-stranded pre-amplification product; and the probe signal detection region (H) on the probe (P) is complementarily paired with the reverse-complementary sequence (h') of the primer signal detection region at 3' end of one single-stranded pre-amplification product (S1) of the double-stranded pre-amplification product, the primer anchoring region (A') on the probe (P) is complementarily paired with the probe anchoring region (A) at 5' end of the single-stranded pre-amplification product (S1), and the 3'end of the single-stranded pre-amplification product (S1) can continue to extend to 5' end of the probe (P), to obtain a partially or completely reverse-complementary sequence of the probe (P), that is, a secondary amplification in which the single-stranded pre-amplification product (S1) is used as a primer and the probe (P) is used as a template is completed, to obtain a secondary amplification double-stranded product formed with the probe (P). At this time, the distance between the first detection group and the second detection group is increased due to a change from the single strand to the double strand, and the efficiency of fluorescence resonance energy transfer is decreased, such that a fluorescence signal change is produced and may be detected by an instrument. After the completion of PCR amplification, when the melting curve analysis is performed, the secondary amplification double-stranded product formed with the probe (P) will melt, such that the probe (P) with the the first detection group and the second detection group becomes a single-stranded state, at this time, the distance between the first detection group and the second detection group will be decreased, and the efficiency of fluorescence resonance energy transfer will be increased, such that a fluorescence signal change is produced again, which may be used for melting curve analysis. When a Tm value at a peak position of the melting curve is detected to be within a range of a characteristic Tm value of the target in the sample to be detected in a specific detection channel, it indicates that there is a target contained in the sample to be detected. The specific detection channel is a fluorescent channel corresponding to the first detection group on the probe (P), and the characteristic Tm value is a melting temperature of the secondary amplification double-stranded product formed with the probe (P).

In some embodiments of the present invention, the sample to be detected is selected from any one of a serum sample, a plasma sample, a whole blood sample, a sputum sample, a swab sample, a lavage fluid sample, a fresh tissue sample, a formalin fixed paraffin embedded tissue.

In some specific embodiments of the present invention, the instrument and reagent consumables used for the PCR amplification in the method may be a SLAN real-time fluorescence quantitative PCR instrument and reagent consumables from Shanghai Hongshi Medical Technology Co., Ltd. In some other specific embodiments of the present invention, for data analysis, the detection results of the present invention may be analyzed by using an analysis software of the SLAN real-time fluorescence quantitative PCR instrument from Shanghai Hongshi Medical Technology Co., Ltd. In the present invention, the target may be selected from at least one of adenovirus (ADV), influenza B virus (IBV), mycoplasma pneumoniae (MP), 2019 severe acute respiratory syndrome coronavirus (SARS-CoV-2), respiratory syncytial virus (RSV), influenza A virus (IAV) and non-small cell lung cancer.

The beneficial effects of the present invention are as follows:
(1) Multiplex detection: the primer probe and the primer probe set described in the present invention can simultaneously analyze the two dimensions of a fluorescence channel and a melting temperature in a single tube reaction, that is, different targets may be detected by using a same fluorescence channel and by means of melting temperature characteristics; or, by using different fluorescent channels, the detection of target species may be achieved by multiplying the number of the fluorescent channels by the melting temperature characteristic;
(2) Low fluorescence background: for each fluorescence channel, only one probe is used, and then a differentiation may be achieved by the different melting temperatures of the amplification products, thereby greatly reducing the fluorescence background in the PCR reaction and improving reaction sensitivity;
(3) adjustable melting temperature: in the detection method by using the primer probe of the present invention, a differentiation is achieved by using the different melting temperatures of the secondary amplification products, such that the melting temperatures of the secondary amplification double-stranded products formed with the probe (P) may be increased or decreased by adjusting a length or sequence of the primer signal detection region (h), or adjusting a position at which the primer signal detection region (h) is reversely complementary with the probe signal detection region (H) of the probe (P);
(4) Good inclusiveness: compared with a Taqman hydrolysis probe method which requires three parts that are paired with the target sequence in a reversely complementary manner, in the primer probe of the present invention, two parts, namely the forward primer (F) and the reverse primer (R) are paired with the target sequence in a reversely complementary manner, such that when there are many high variation regions in the target sequence (such as viruses or bacterial genomes), the primer probe of the present invention has a better inclusiveness and a lower design difficulty;
(5) Low cost: by means of the method of the present invention, a detection may be completed in a single tube reaction by only requiring a fluorescence quantitative PCR instrument, without the need for additional downstream instruments and without the need for chips or multiple reaction tubes for dividing reactions, thereby greatly reducing the cost of instruments and consumables, meanhwile, the number of fluorescence probes in the reagent is reduced, thereby greatly reducing the cost of the reagent, and allowing a large-scale promotion to be possible;
(6) Single tube reaction: the method has low sample consumption, is particularly suitable for the detection of a rare sample, and can greatly increase a concentration of the sample added to the detection reaction, so as to improve detection sensitivity, and provide the possibility for a subsequent extraction-free one-tube detection;
(7) Easy and simple operation: only a fluorescence quantitative PCR instrument is required for detection, without subsequent steps such as for capillary electrophoresis or nucleic acid hybridization, etc., meanwhile, the preparation is simple, and there is no need to operate after the instrument is enabled;
(8) No easy to cause pollution: in the method described in the present invention, the instrument is completely closed after the sample addition is completed, without the need to open the cover for subsequent detection, thereby greatly reducing the possibility of polluting an experimental environment;
(9) High sensitivity: the method described in the present invention has a very low requirement for the length of the target sequence, when the target type is a short fragment nucleic acid, such as a free nucleic acid, a higher sensitivity is achieved in detection for a shorter target sequence length; and
(10) Wide applicable range: the method described in the present invention is suitable for nucleic acid detection of various sample types, including a serum sample, a plasma sample, a whole blood sample, a sputum sample, a swab sample, a lavage fluid sample, a fresh tissue sample, a formalin fixed paraffin embedded tissue (FFPE).

### Brief Description of the Drawings

The present invention will be further illustrated in combination with the accompanying drawings.
Fig. 1 shows a melting curve representing RSV (82.23°C) positive in a VIC channel according to a singleplex PCR detection reaction of Example 1.
Fig. 2 shows a melting curve representing RSV (74.98°C) positive in a VIC channel according to a singleplex PCR detection reaction of Example 1.
Fig. 3 shows a melting curve representing RSV (68.25°C) positive in a VIC channel according to a singleplex PCR detection reaction of Example 1.
Fig. 4 shows a melting curve representing RSV (72.18°C) positive in a CY5 channel according to a singleplex PCR detection reaction of Example 2.
Fig. 5 shows a melting curve representing RSV (73.49°C) positive in a CY5 channel according to a singleplex PCR detection reaction of Example 3.

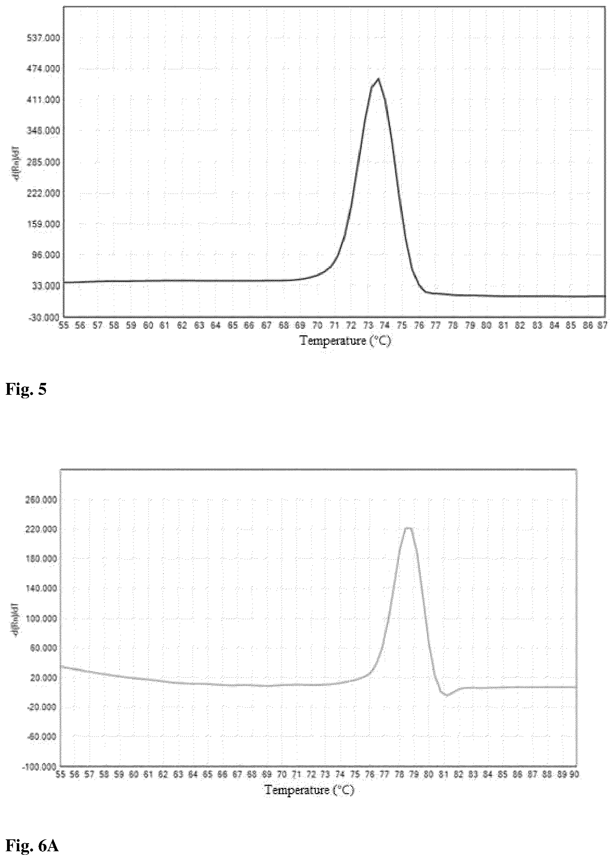

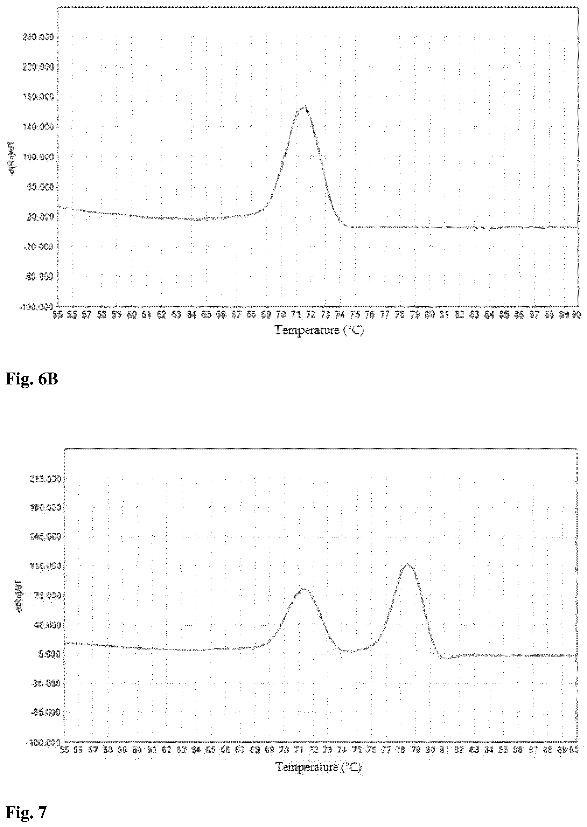

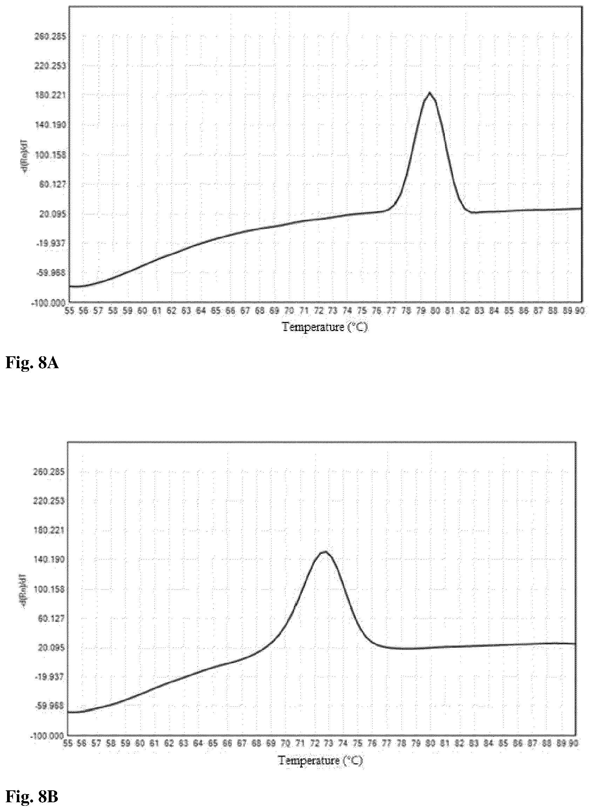

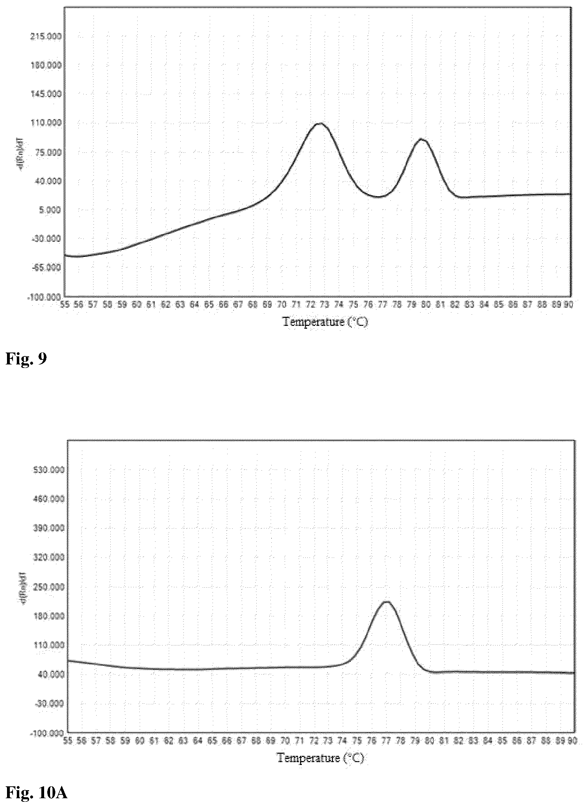

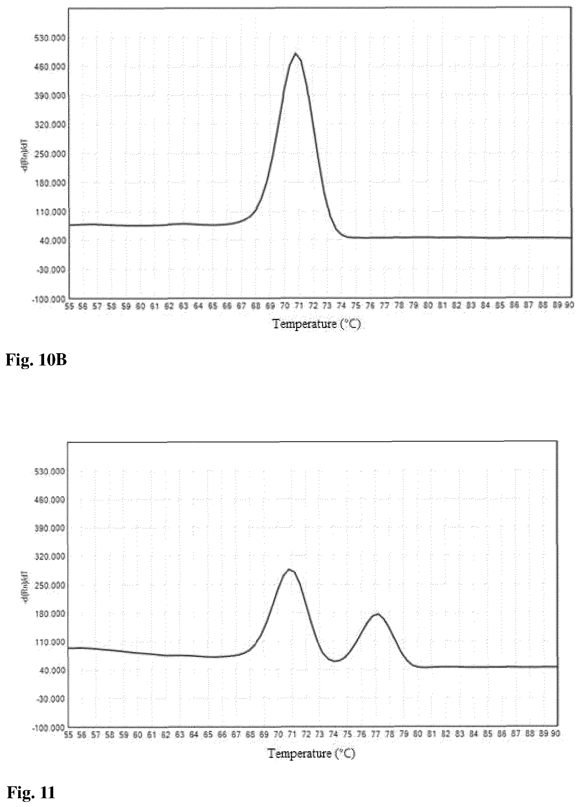

Fig. 6-11 are views of melting curves of individual respiratory virus positive samples according to multiplex PCR in Example 4, wherein, Fig. 6A shows that an ADV (78.36°C) positive melting peak appears in a reaction well with addition of an ADV positive template in a ROX channel; Fig. 6B shows that an IBV (71.30°C) positive melting peak appears in a reaction well with addition of an IBV positive template in a ROX channel; Fig. 7 shows that both an ADV (78.36°C) melting peak and an IBV (71.30°C) melting peak simultaneously appear in a reaction well with addition of both an ADV positive template and an IBV positive template in a ROX channel; Fig. 8A shows that an MP (79.57°C) positive melting peak appears in a reaction well with addition of an MP positive template in an FAM channel; Fig. 8B shows that a SARS-CoV-2 (72.65°C) positive melting peak appears in a reaction well with addition of a SARS-CoV-2 positive template in an FAM channel; Fig. 9 shows that dual melting peaks of MP (79.57°C) and SARS-CoV-2 (72.65°C) simultaneously appear in a reaction well with addition of an MP positive template and a SARS-CoV-2 positive template in an FAM channel; Fig. 10A shows that an IAV (76.98°C) positive melting peak appears in a reaction well with addition of an IAV positive template in a CY5 channel; Fig. 10B shows that an RSV (70.84°C) positive melting peak appears in a reaction well with addition of a RSV positive template in a CY5 channel; and Fig. 11 shows that dual melting peaks of IAV (76.98°C) and RSV (70.84°C) simultaneously appear in a reaction well with addition of both an IAV positive template and a RSV positive template in a CY5 channel. It may be seen that, when the method of the present invention is used to detect six pathogens, a good specificity is achieved, the melting curve has a single pattern, and the Tm values of two positive targets in a same channel may be clearly differentiated.
Fig. 12 shows a melting curve representing RSV (82.23°C) positive and primer dimer (without melting peak) using probe P1 in a VIC channel according to a singleplex PCR detection reaction of comparative example;
Fig. 13 shows a melting curve representing RSV positive (82.23°C, a melting peak with a high Rm value) and primer dimer (68.52°C, a melting peak with a low Rm value) using probe P2 in an FAM channel according to a singleplex PCR detection reaction of comparative example.
Fig. 14 is a melting curve of a singleplex PCR non small cell lung cancer EGFR exon 20 H773_V774insH mutation positive sample amplified by a reverse primer (R) with a signal detection region in Example 5.
Fig. 15 is a melting curve of a singleplex PCR non small cell lung cancer EGFR exon 20 H773_V774insH mutation positive sample amplified by a reverse primer (R) with a signal detection region in Example 6.
Fig. 16 is a melting curves of positive samples of IMP mutation in genes resistant to carbapenems, OXA-48 mutation in genes resistant to carbapenems, KPC mutation in genes resistant to carbapenems, and VIM mutation in genes resistant to carbapenems according to multiplex PCR detections in Example 7.
Fig. 17 is a melting curve of melting peaks of a non small cell lung cancer EGFR exon 21 L861Q mutation positive sample according to a singleplex PCR detection reaction in an HEX channel in Example 8.

### Detailed Description of the Embodiments

In order to make the present invention easier to understand, the present invention will be further illustrated in combination with examples. These examples are only for illustration and are not intended to limite the applicable scope of the present invention. Unless otherwise specified, the raw materials or components used in the present invention may be obtained via commercial approaches or conventional methods.

### Example 1: Singleplex PCR detection of respiratory syncytial virus (RSV)

(1) Base sequences of a primer probe used for Singleplex PCR detection of respiratory syncytial virus (RSV) are shown in Table 1.

**Table 1: Base sequences of primer probe**

| Name of primer probe | Number | Nucleotide sequence (5'-3') | Label |
|---|---|---|---|
| P1-1 | SEQ ID NO:1 | CCCTGGGAGCTCATGGAATCGCTG GTCCTGTCGACGCTGCGCACACTG GGACGGCACCG | T14-VIC, T26-BHQ1 3' Spacer C3 |
| R1-1 | SEQ ID NO:2 | CGGCACCGAAATATTGCAGATGTG ACACCTTAGTA | / |
| R2-1 | SEQ ID NO:3 | CGACGCTGCGAAATATTGCAGATG TGACACCTTAGTA | / |
| R3-1 | SEQ ID NO:4 | CTGGTCCTGTAAATATTGCAGATGT GACACCTTAGTA | / |
| F1-1 | SEQ ID NO:5 | GCTCCCAGGGTTTAACCTGGACTA AGAAGAGGAACGACAGAT | / |

In the above primer probe:
the forward primer F1-1 (SEQ ID NO: 5) is a specific primer designed for a target sequence of respiratory syncytial virus, and the F1-1 has a total length of 42 bp, wherein a 1^{st} to 29^{th} bases at 3' end thereof serve as a target sequence binding region 1, and a 1^{st} to 10^{th} bases at 5' end thereof serve as a probe anchoring region (A), which is reversely complementary with a sequence of a 1^{st} to 10^{th} bases (i.e. a primer anchoring region (A')) at 5' end of the probe P1-1 (SEQ ID NO: 1);
the reverse primer R1-1 (SEQ ID NO: 2) is a specific primer designed for the target sequence of respiratory syncytial virus, and the R1-1 has a total length of 35 bp, wherein a 1^{st} to 24^{th} bases at 3' end thereof serve as a target sequence binding region 2, and a 1^{st} to 8^{th} bases at 5' end thereof serve as a primer signal detection region (h1), which is identical to a sequence of a 1^{st} to 8^{th} bases at 3' end of the probe P1-1 (SEQ ID NO: 1);
the reverse primer R2-1 (SEQ ID NO: 3) is a specific primer designed for the target sequence of respiratory syncytial virus, and the R2-1 has a total length of 37 bp, wherein a 1^{st} to 24^{th} bases at 3' end thereof serve as a target sequence binding region 2, and a 1^{st} to 10^{th} bases at 5' end thereof serve as a primer signal detection region (h2), which is identical to a sequence of a 19^{th} to 28^{th} bases at 3'end of the probe P1-1 (SEQ ID NO: 1); and
the reverse primer R3-1 (SEQ ID NO: 4) is a specific primer designed for the target sequence of respiratory syncytial virus, and the R3-1 has a total length of 37 bp, wherein a 1^{st} to 24^{th} bases at 3' end thereof serve as a target sequence binding region 2, and a 1^{st} to 10^{th} bases at 5' end thereof serve as a primer signal detection region (h), which is identical to a sequence of a 29^{th} to 38^{th} bases at 3'end of the probe P1-1 (SEQ ID NO: 1).

(2) A singleplex PCR detection for respiratory syncytial virus (RSV) was performed by using the above primer probe.

A reaction system used for detection was shown in Table 2.

**Table 2: Singleplex PCR detection reaction system for respiratory syncytial virus**

| Reagents and components | Concentration |
|---|---|
| 2× PCR Reaction buffer | 1× |
| DNA polymerase | 2U |
| Reverse transcriptase | 5U |
| Forward primer (F 1 -1) | 500 nM |
| Reverse primers (R1-1, R2-1, or R3-1) | 100 nM |
| Probe (P1-1) | 400 nM |
| Nucleic acid template | 30 copies |
| Ultra-pure water | Adding to 20 µL |

| | |
|---|---|
| Note: The 2xPCR reaction buffer comprises 3 mM MgCl₂, 30 mM Tris-HCl buffer with a pH of 8.3, 0.5 mM dNTP, and 70 mM (NH₄)₂SO₄. | |

Nucleic acid template: an in-vitro transcribed RNA of the target sequence of respiratory syncytial virus was used as the nucleic acid template, and the reverse primers (R) with different signal detection region sequences were used to separately detect the target sequence of respiratory syncytial virus respectively, to verify a detection ability for single virus infection. The ultra-pure water was used as a no template control (NTC).

### Reaction preparation:

After the preparation of the nucleic acid template was completed, the reaction system was prepared according to the ratios described in Table 2.

### PCR amplification and melting curve analysis:

After a PCR tube was sealed by a cap, the sample was gently and uniformly mixed, and centrifuged for a short time and then left for 5 min at a room temperature. The PCR tube was put in a handheld centrifuge again, centrifuged for a short time, and then transferred to a tray of SLAN real-time fluorescence quantitative PCR instrument from Shanghai Hongshi Medical Technology Co., Ltd. The procedure used was as follows: performing reverse transcription at 55 °C for 15 minutes; performing pre-denaturation at 95 °C for 3 minutes; performing denaturation at 94 °C for 10 seconds, and performing annealing and extension at 56 °C for 30 seconds, for a total of 45 cycles, without lighting; performing denaturation at 94 °C for 150 seconds, and perfroming incubation at a constant temperature of 40°C for 300 seconds; and performing melting curve analysis at 40-85°C, with a heating rate of 0.05°C/s and with lighting, to obtain a melting curve.

### Data analysis:

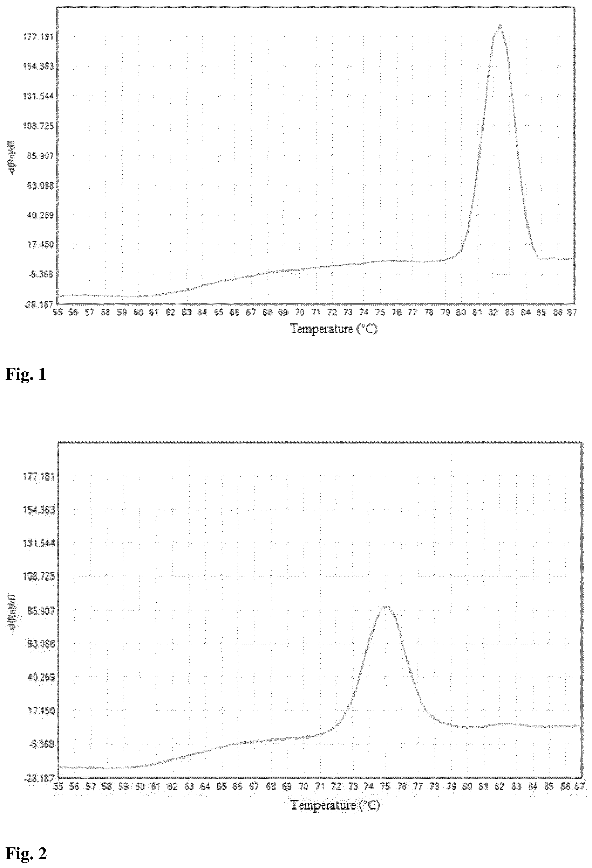

The results were interpreted via Tm values of the melting curves by using an analysis software of the SLAN real-time fluorescence quantitative PCR instrument from Shanghai Hongshi Medical Technology Co., Ltd. Figs. 1-3 are the melting curves of singleplex PCR respiratory syncytial virus positive samples amplified by the reverse primers (R) with different signal detection regions.

It may be seen from Figs. 1-3 that, when the primer probe of the present invention was used, an increase or decrease of the melting temperature of the amplification double-stranded product formed with the probe (P) may be achieved by adjusting a length or sequence of the primer signal detection region (h) of the reverse primer (R), and accordingly adjusting a position at which the reverse-complementary sequence (h') of the primer signal detection region (h) is recersely complementary with the probe signal detection region (H) of the probe (P).

### Example 2: Singleplex PCR detection of respiratory syncytial virus (RSV)

(1) Base sequences of a primer probe used for Singleplex PCR detection of respiratory syncytial virus (RSV) are shown in Table 3.

**Table 3: Base sequences of primer probe**

| Name of primer probe | Number | Nucleotide sequence (5'-3') | Label |
|---|---|---|---|
| P1-1 | SEQ ID NO:1 | CCCTGGGAGCTCATGGAATCGCTG GTCCTGTCGACGCTGCGCACACTG GGACGGCACCG | T14-CY5, T26-BHQ1 3' Spacer C3 |
| R1-2 | SEQ ID NO:6 | CGACGCTGAAATATTGCAGATGTG ACACCTTAGTA | / |
| F1-2 | SEQ ID NO:7 | CGGTGCCGTCCCTTTAACCTGGAC TAAGAAGAGGAACGACAGAT | / |

In the above primer probe:
the forward primer F1-1 (SEQ ID NO: 7) is a specific primer designed for a target sequence of respiratory syncytial virus, and the F1-2 has a total length of 44 bp, wherein a 1^{st} to 29^{th} bases at 3' end thereof serve as a target sequence binding region 1, and a 1^{st} to 12^{th} bases at 5' end thereof serve as a probe anchoring region (A), which is reversely complementary with a sequence of a 1^{st} to 12^{th} bases (i.e. a primer anchoring region (A')) at 3' end of the probe P1-1 (SEQ ID NO: 1); and
the reverse primer R1-1 (SEQ ID NO: 6) is a specific primer designed for the target sequence of respiratory syncytial virus, and the R1-2 has a total length of 35 bp, wherein a 1^{st} to 24^{th} bases at 3' end thereof serve as a target sequence binding region 2, and a 1^{st} to 8^{th} bases at 5' end thereof serve as a primer signal detection region (h), which is identical to a sequence of a 21^{st} to 28^{th} bases at 3' end of the probe P1-1 (SEQ ID NO: 1).

In the above primer probe, the forward primer F1-2 and the reverse primer R1-2 specifically amplify the target (respiratory syncytial virus), and after amplification, one single-stranded pre-amplification product of the formed double-stranded pre-amplification product comprises, from 5' end to 3' end, the probe anchoring region (A), the target sequence, and a reverse-complementary sequence (h') of the primer signal detection region. The probe anchoring region (A) and the reverse-complementary sequence (h') of the single-stranded pre-amplification product are complementarily paired with the primer anchoring region (A') and the primer signal detection region (h) of the probe P1-1 respectively, forming a structure similar to "Omega".

(2) A singleplex PCR detection for respiratory syncytial virus (RSV) was performed by using the above primer probe.

A reaction system used for detection was shown in Table 4.

**Table 4: Singleplex PCR detection reaction system for respiratory syncytial virus**

| Reagents and components | Concentration |
|---|---|
| 2 × PCR reaction buffer | 1× |
| DNA polymerase | 2U |
| Reverse transcriptase | 5U |
| Forward primer (F1-2) | 500 nM |
| Reverse primer (R1-2) | 100 nM |
| Probe (P1-2) | 400 nM |
| Nucleic acid template | 30 copies |
| Ultra pure water | Adding to 20 µL |

| | |
|---|---|
| Note: The 2xPCR reaction buffer comprises 3 mM MgCl₂, 30 mM Tris-HCl buffer with a pH of 8.3, 0.5 mM dNTP, and 70 mM (NH₄)₂SO₄. | |

Nucleic acid template: an in-vitro transcribed RNA of the target sequence of respiratory syncytial virus was used as the nucleic acid template. The ultra-pure water was used as a no template control (NTC).

### Reaction preparation:

After the preparation of the nucleic acid template was completed, the reaction system was prepared according to the ratios described in Table 4.

### PCR amplification and melting curve analysis:

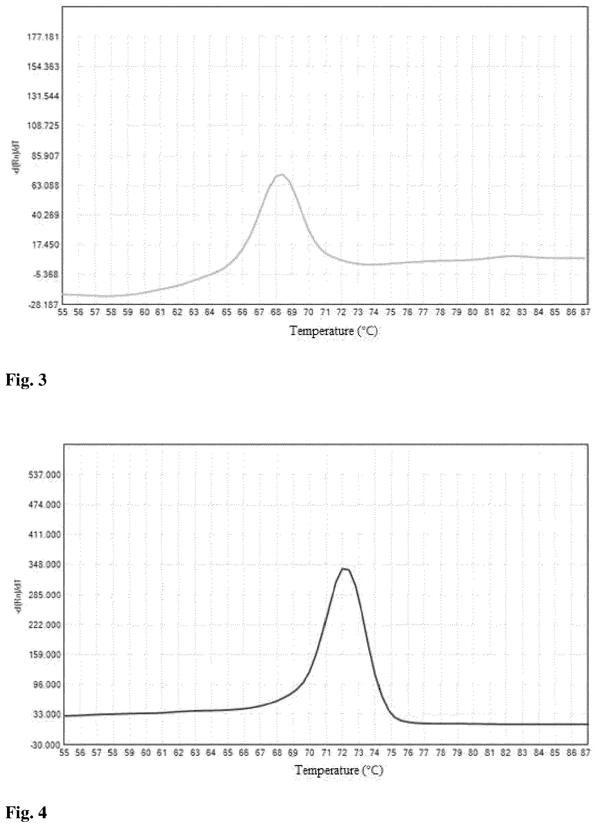

After a PCR tube was sealed by a cap, the sample was gently and uniformly mixed, and centrifuged for a short time and then left for 5 min at a room temperature. The PCR tube was put in a handheld centrifuge again, centrifuged for a short time, and then transferred to a tray of SLAN real-time fluorescence quantitative PCR instrument from Shanghai Hongshi Medical Technology Co., Ltd. The procedure used was as follows: performing reverse transcription at 55 °C for 15 minutes; performing pre-denaturation at 95°C for 3 minutes; performing denaturation at 94 °C for 10 seconds, and performing annealing and extension at 56 °C for 30 seconds, for a total of 45 cycles, without lighting; performing denaturation at 94 °C for 150 seconds, and perfroming incubation at a constant temperature of 40°C for 300 seconds; and performing melting curve analysis at 40-85°C, with a heating rate of 0.05°C/s and with lighting, to obtain a melting curve, as shown in Fig. 4.

### Data analysis:

The results were interpreted via a Tm value of the melting curve by using an analysis software of the SLAN real-time fluorescence quantitative PCR instrument from Shanghai Hongshi Medical Technology Co., Ltd.

It may be seen from Fig. 4 that, when the primer probe design method of the present invention was used, a target may be clearly detected by adopting PCR amplification and melting curve analysis.

### Example 3: Singleplex PCR detection of respiratory syncytial virus (RSV)

(1) Base sequences of a primer probe used for Singleplex PCR detection of respiratory syncytial virus (RSV) are shown in Table 5.

**Table 5: Base sequences of primer probe**

| Name of primer probe | Number | Nucleotide sequence (5'-3') | Label |
|---|---|---|---|
| P1-1 | SEQ ID NO:1 | CCCTGGGAGCTCATGGAATCGCTG GTCCTGTCGACGCTGCGCACACTG GGACGGCACCG | T14-CY5, T26-BHQ1 3' Spacer C3 |
| R1-3 | SEQ ID NO:8 | CGACGCTGCTTTGGCATCGTATTGC AGATGTGACACCTTAGTA | / |
| F1-3 | SEQ ID NO:9 | CGGTGCCGTCCCAAAGGCATCGAA CCTGGACTAAGAAGAGGAACGAC AGAT | / |

In the above primer probe:
the forward primer F1-3 (SEQ ID NO: 9) is a specific primer designed for a target sequence of respiratory syncytial virus, and the F1-3 has a total length of 51 bp, wherein a 1^{st} to 29^{th} bases at 3' end thereof serve as a target sequence binding region 1, and a 1^{st} to 10^{th} bases at 5' end thereof serve as a probe anchoring region (A), which is reversely complementary with a sequence of a 1^{st} to 10^{th} bases (i.e. a primer anchoring region (A')) at 3' end of the probe P1-1 (SEQ ID NO: 1); and
the reverse primer R1-3 (SEQ ID NO: 8) is a specific primer designed for the target sequence of respiratory syncytial virus, and the R1-3 has a total length of 43 bp, wherein a 1^{st} to 24^{th} bases at 3' end thereof serve as a target sequence binding region 2, and a 1^{st} to 9^{th} bases at 5' end thereof serve as a primer signal detection region (h), which is identical to a sequence of a 20^{th} to 28^{th} bases at 3' end of the probe P1-1 (SEQ ID NO: 1).

In the above primer probe, the forward primer F1-3 and the reverse primer R1-3 specifically amplify the target (respiratory syncytial virus), and after amplification, one single-stranded pre-amplification product of the formed double-stranded pre-amplification product comprises, from 5' end to 3' end, the probe anchoring region (A), the target sequence, and the reverse-complementary sequence (h') of the primer signal detection region. The probe anchoring region (A) and the reverse- sequence (h') of the single-stranded pre-amplification product are complementarily paired with the primer anchoring region (A') and the primer signal detection region (h) of the probe P1-1 respectively, forming a structure similar to a "stem".

(2) A singleplex PCR detection for respiratory syncytial virus (RSV) was performed by using the above primer probe.

A reaction system used for detection was shown in Table 6.

**Table 6: Singleplex PCR detection reaction system for respiratory syncytial virus**

| Reagents and components | Concentration |
|---|---|
| 2 × PCR reaction buffer | 1× |
| DNA polymerase | 2U |
| Reverse transcriptase | 5U |
| Forward primer (F1-3) | 500 nM |
| Reverse primer (R1-3) | 100 nM |
| Probe (P1-1) | 400 nM |
| Nucleic acid template | 30 copies |
| Ultra pure water | Adding to 20 µL |

| | |
|---|---|
| Note: The 2xPCR reaction buffer comprises 3 mM MgCl₂, 30 mM Tris-HCl buffer with a pH of 8.3, 0.5 mM dNTP, and 70 mM (NH₄)₂SO₄. | |

Nucleic acid template: an in-vitro transcribed RNA of the target sequence of respiratory syncytial virus was used as the nucleic acid template. The ultra-pure water was used as a no template control (NTC).

### Reaction preparation:

After the preparation of the nucleic acid template was completed, the reaction system was prepared according to the ratios described in Table 6.

### PCR amplification and melting curve analysis:

After a PCR tube was sealed by a cap, the sample was gently and uniformly mixed, and centrifuged for a short time and then left for 5 min at a room temperature. The PCR tube was put in a handheld centrifuge again, centrifuged for a short time, and then transferred to a tray of SLAN real-time fluorescence quantitative PCR instrument from Shanghai Hongshi Medical Technology Co., Ltd. The procedure used was as follows: performing reverse transcription at 55 °C for 15 minutes; performing pre-denaturation at 95°C for 3 minutes; performing denaturation at 94 °C for 10 seconds, and performing annealing and extension at 56 °C for 30 seconds, for a total of 45 cycles, without lighting; performing denaturation at 94 °C for 150 seconds, and perfroming incubation at a constant temperature of 40°C for 300 seconds; and performing melting curve analysis at 40-85°C, with a heating rate of 0.05°C/s and with lighting, to obtain a melting curve, as shown in Fig. 5.

### Data analysis:

The results were interpreted via a Tm value of the melting curve by using an analysis software of the SLAN real-time fluorescence quantitative PCR instrument from Shanghai Hongshi Medical Technology Co., Ltd.

It may be seen from Fig. 5 that, when the primer probe design method of the present invention was used, even if the primary amplification product formed a "stem" structure, the probe anchoring region (A) and the reverse-complementary sequence (h') of primer signal detection region (H') were not masked, such that a target may be clearly detected.

### Example 4: Multiplex PCR detection for influenza A virus (IAV), influenza B virus (IBV), respiratory syncytial virus (RSV), adenovirus (ADV), mycoplasma pneumoniae (MP) and 2019 severe acute respiratory syndrome coronavirus (SARS-CoV-2)

(1) Base sequences of a primer probe used for multiplex PCR detection for influenza A virus (IAV), influenza B virus (IBV), respiratory syncytial virus (RSV), adenovirus (ADV), mycoplasma pneumoniae (MP) and 2019 severe acute respiratory syndrome coronavirus (SARS-CoV-2) are shown in Table 7.

**Table 7: Base sequences of primer probe**

| Name of primer probe | Number | Nucleotide sequence (5'-3') | Label |
|---|---|---|---|
| P1-4 | SEQ ID NO:10 | TGCCAGGTCCTATTGTCACGATCG TTAGCCGTGGTCCGCACGTCAGAG CGAGTCCGAGG | T16 ROX, T26BHQ2 3' Spacer C3 |
| P2-4 | SEQ ID NO:11 | TCGCAAGTGAGGCACTACGTGAGC GATGGACGGAGGCAGGAGCTGTG GGACCAGGTGCG | T16 FAM T27 BHQ1 3' Spacer C3 |
| P3-4 | SEQ ID NO:12 | GACTCGAGCCTCATGAAGCCGACA CTGTCCGTGGAGCGACTCGACCCT GCGACCTGGCA | T14CY5 T26BHQ2 3' Spacer C3 |
| F1-4 | SEQ ID NO:13 | GGACCTGGCAAAATGCAACATGA CCAAGGACTGG | / |
| R1-4 | SEQ ID NO:14 | GTCCGAGGAAATGTAGAAGCCCTG GTAGC | / |
| F2-4 | SEQ ID NO:15 | GGACCTGGCAAACTAAGCAGACTC ACAGAACT | / |
| R2-4 | SEQ ID NO:16 | GGTCCGCAAAAATGAGAACTTGCC ATAGGTT | / |
| F3-4 | SEQ ID NO:17 | GCCTCACTTGCGAAAATAACTCAA GGCGGACGATT | / |
| R3-4 | SEQ ID NO:18 | CCAGGTGCGAAATTAACCACGGCG TTCAGG | / |
| F4-4 | SEQ ID NO:19 | GCCTCACTTGCGAAATGGACAACA GCAGACAAC | / |
| R4-4 | SEQ ID NO:20 | CGATGGACAAATCCACACGTACAA GGTATCT | / |
| F5-4 | SEQ ID NO:21 | GGCTCGAGTCAAAATAATCAGGCA CGAGAACAG | / |
| R5-4 | SEQ ID NO:22 | CGACCTGGCAAAAGACTAGCAAC CTCCATGGC | / |
| F6-4 | SEQ ID NO:23 | GGCTCGAGTCAAAAAGCAACCAA CAACATTCATTG | / |
| R6-4 | SEQ ID NO:24 | CCGACACTGTAAAGCAATTCAGCA TCACAGAC | / |

In the above primer probe:
Both of the forward primer F1-4 (SEQ ID NO: 13) and the reverse primer R1-4 (SEQ ID NO: 14) are specific primers designed for a target sequence of adenovirus. The F1-4 has a total length of 34 bp, wherein a 1^{st} to 21^{st} bases at 3' end thereof serve as a target sequence binding region 1, and a 1^{st} to 10^{th} bases at 5' end thereof serve as a probe anchoring region (A1), which is reversely complementary with a sequence of a 1^{st} to 10^{th} bases (i.e. a primer anchoring region (A1')) of the probe P1-4 (SEQ ID NO: 10). The R1-4 has a total length of 29 bp, wherein a 1^{st} to 19^{th} bases at 3' end thereof serve as a target sequence binding region 2, and a 1^{st} to 8^{th} bases at 5' end thereof serve as a primer signal detection region (h1), which is identical to a sequence of a 1^{st} to 8^{th} bases at 3' end of the probe P1-4 (SEQ ID NO: 10).
Both of the forward primer F2-4 (SEQ ID NO: 15) and the reverse primer R2-4 (SEQ ID NO: 16) are specific primers designed for a target sequence of influenza B virus. The F2-4 has a total length of 32 bp, wherein a 1^{st} to 20^{th} bases at 3' end thereof serve as a target sequence binding region 1, and a 1^{st} to 10^{th} bases at 5' end thereof serve as a probe anchoring region (A1), which is reversely complementary with a sequence of a 1^{st} to 10^{th} bases (i.e. a primer anchoring region (A1')) of the probe P1-4 (SEQ ID NO: 10). The R2-4 has a total length of 31 bp, wherein a 1^{st} to 20^{th} bases at 3' end thereof serve as a target sequence binding region 2, and a 1^{st} to 8^{th} bases at 5' end thereof serve as a primer signal detection region (h2), which is identical to a sequence of a 21^{st} to 28^{th} bases at 3' end of the probe P1-4 (SEQ ID NO: 10).
Both of the forward primer F3-4 (SEQ ID NO: 17) and the reverse primer R3-4 (SEQ ID NO: 18) are specific primers designed for a target sequence of mycoplasma pneumoniae. The F3-4 has a total length of 35 bp, wherein a 1^{st} to 19^{th} bases at 3' end thereof serve as a target sequence binding region 1, and a 1^{st} to 13^{rd} bases at 5' end thereof serve as a probe anchoring region (A2), which is reversely complementary with a sequence of a 1^{st} to 13^{rd} bases (i.e. a primer anchoring region (A2')) of the probe P2-4 (SEQ ID NO: 11). The R3-4 has a total length of 30 bp, wherein a 1^{st} to 18^{th} bases at 3' end thereof serve as a target sequence binding region 2, and a 1^{st} to 9^{th} bases at 5' end thereof serve as a primer signal detection region (h3), which is identical to a sequence of a 1^{st} to 9^{th} bases at 3' end of the probe P2-4 (SEQ ID NO: 11).
Both of the forward primer F4-4 (SEQ ID NO: 19) and the reverse primer R4-4 (SEQ ID NO: 20) are specific primers designed for a target sequence of 2019 severe acute respiratory syndrome coronavirus. The F4-4 has a total length of 33 bp, wherein a 1^{st} to 19^{th} bases at 3' end thereof serve as a target sequence binding region 1, and a 1^{st} to 13^{rd} bases at 5' end thereof serve as a probe anchoring region (A2), which is reversely complementary with a sequence of a 1^{st} to 13^{rd} bases (i.e. a primer anchoring region (A2')) of the probe P2-4 (SEQ ID NO: 11). The R4-4 has a total length of 31 bp, wherein a 1^{st} to 20^{th} bases at 3' end thereof serve as a target sequence binding region 2, and a 1^{st} to 8^{th} bases at 5' end thereof serve as a primer signal detection region (h4), which is identical to a sequence of a 29^{th} to 36^{th} bases at 3' end of the probe P2-4 (SEQ ID NO: 11).
Both of the forward primer F5-4 (SEQ ID NO: 21) and the reverse primer R4-4 (SEQ ID NO: 22) are specific primers designed for a target sequence of influenza A virus (IAV). The F5-4 has a total length of 33 bp, wherein a 1^{st} to 20^{th} bases at 3' end thereof serve as a target sequence binding region 1, and a 1^{st} to 10^{th} bases at 5' end thereof serve as a probe anchoring region (A3), which is reversely complementary with a sequence of a 1^{st} to 10^{th} bases (i.e. a primer anchoring region (A3')) of the probe P3-4 (SEQ ID NO: 12). The R5-4 has a total length of 32 bp, wherein a 1^{st} to 19^{th} bases at 3' end thereof serve as a target sequence binding region 2, and a 1^{st} to 10^{th} bases at 5' end thereof serve as a primer signal detection region (h5), which is identical to a sequence of a 1^{st} to 10^{th} bases at 3' end of the probe P3-4 (SEQ ID NO: 12).
Both of the forward primer F6-4 (SEQ ID NO: 23) and the reverse primer R6-4 (SEQ ID NO: 24) are specific primers designed for a target sequence of respiratory syncytial virus. The F6-4 has a total length of 35 bp, wherein a 1^{st} to 22^{nd} bases at 3' end thereof serve as a target sequence binding region 1, and a 1^{st} to 10^{th} bases at 5' end thereof serve as a probe anchoring region (A3), which is reversely complementary with a sequence of a 1^{st} to 10^{th} bases (i.e. a primer anchoring region (A3')) of the probe P3-4 (SEQ ID NO: 12). The R6-4 has a total length of 32 bp, wherein a 1^{st} to 19^{th} bases at 3' end thereof serve as a target sequence binding region 2, and a 1^{st} to 10^{th} bases at 5' end thereof serve as a primer signal detection region (h6), which is identical to a sequence of a 32^{nd} to 41^{st} bases at 3' end of the probe P3-4 (SEQ ID NO: 12).

The characteristic Tm values of the above probes are shown in Table 8:

**Table 8**

| Fluorescent channel | Melting temperature (Tm, °C) | | | |
|---|---|---|---|---|
| | 66≤Tm<74 | 69≤Tm<75 | 72≤Tm<78 | 76≤Tm<84 |
| ROX | / | / | IBV | ADV |
| FAM | / | SARS-CoV-2 | / | MP |
| Cy5 | RSV | / | / | IAV |

(2) A multiplex PCR detection for influenza A virus (IAV), influenza B virus (IBV), respiratory syncytial virus (RSV), adenovirus (ADV), mycoplasma pneumoniae (MP) and 2019 severe acute respiratory syndrome coronavirus (SARS-CoV-2) was performed by using the above primer probe. The reaction system used for detection was shown in Table 9.

**Table 9: Multiple PCR detection reaction system for influenza A virus (IAV), influenza B virus (IBV), respiratory syncytial virus (RSV), adenovirus (ADV), mycoplasma pneumoniae (MP) and 2019 severe acute respiratory syndrome coronavirus (SARS-CoV-2)**

| Reagents and components | Concentration |
|---|---|
| 2× PCR Reaction Buffer | 1× |
| DNA Polymerase | 2U |
| Reverse transcriptase | 5U |
| Forward primer (F1-4) | 500 nM |
| Reverse primer (R1-4) | 100 nM |
| Probe (P1-4) | 400 nM |
| Forward primer (F2-4) | 500 nM |
| Reverse primer (R2-4) | 100 nM |
| Forward primer (F3-4) | 500 nM |
| Reverse primer (R3-4) | 100 nM |
| Probe (P2-4) | 400 nM |
| Forward primer (F4-4) | 500 nM |
| Reverse primer (R4-4) | 100 nM |
| Forward primer (F5-4) | 500 nM |
| Reverse primer (R5-4) | 100 nM |
| Probe (P3-4) | 400 nM |
| Forward primer (F6-4) | 500 nM |
| Reverse primer (R6-4) | 100 nM |
| Nucleic acid template | 30 copies |
| Ultra pure water | Adding to 20 µL |

| | |
|---|---|
| Note: The 2xPCR reaction buffer comprises 3 mM MgCl₂, 30 mM Tris-HCl buffer with a pH of 8.3, 0.5 mM dNTP, and 70 mM (NH₄)₂SO₄. | |

Nucleic acid template: an in-vitro transcribed RNA of the target sequence of the influenza A virus, an in-vitro transcribed RNA of the target sequence of the influenza B virus, an in-vitro transcribed RNA of the target sequence of the respiratory syncytial virus, an in-vitro transcribed RNA of the target sequence of the adenovirus, an in-vitro transcribed RNA of the target sequence of the mycoplasma pneumoniae and an in-vitro transcribed RNA of the target sequence of the 2019 severe acute respiratory syndrome coronavirus (SARS-CoV-2) were used as the nucleic acid templates, and the two target sequence templates of a same fluorescent channel were mixed for detection, to verify a detection ability for mixed virus infection. The ultra-pure water was used as a no template control (NTC).

### Reaction preparation:

After the preparation of the nucleic acid templates was completed, the reaction system was prepared according to the ratios described in Table 9.

### PCR amplification and melting curve analysis:

After a PCR tube was sealed by a cap, the sample was gently and uniformly mixed, and centrifuged for a short time and then left for 5 min at a room temperature. The PCR tube was put in a handheld centrifuge again, centrifuged for a short time, and then transferred to a tray of SLAN real-time fluorescence quantitative PCR instrument from Shanghai Hongshi Medical Technology Co., Ltd. The procedure used was as follows: performing reverse transcription at 55 °C for 15 minutes; performing pre-denaturation at 95°C for 3 minutes; performing denaturation at 94 °C for 10 seconds, and performing annealing and extension at 56 °C for 30 seconds, for a total of 45 cycles, without lighting; performing denaturation at 94 °C for 150 seconds, and perfroming incubation at a constant temperature of 40°C for 300 seconds; and performing melting curve analysis at 40-85°C, with a heating rate of 0.05°C/s and with lighting, to obtain melting curves, as shown in Figs. 6-11 respectively.

### Data analysis:

The results were interpreted via Tm values of the melting curves by using an analysis software of the SLAN real-time fluorescence quantitative PCR instrument from Shanghai Hongshi Medical Technology Co., Ltd.

Fig. 6A and Fig. 6B show that an ADV (78.36°C) melting peak and an IDV (71.30°C) melting peak appear in a PCR reaction well with addition of an ADV positive template and an IDV positive template in a ROX channel, respectively.

Fig. 7 shows that dual melting peaks of ADV (78.36°C) and IDV (71.30°C) simultaneously appear in a PCR reaction well with addition of both an ADV positive template and an IDV positive template in a ROX channel.

Fig. 8A and Fig. 8B show that an MP (79.57°C) melting peak and a SARS-CoV-2 (72.65°C) melting peak appear in a PCR reaction well with addition of an MP positive template and a SARS-CoV-2 positive template in an FAM channel, respectively.

Fig. 9 shows that dual melting peaks of MP (79.57°C) and SARS-CoV-2 (72.65°C) simultaneously appear in a PCR reaction well with addition of both an MP positive template and a SARS-CoV-2 positive template in an FAM channel.

Fig. 10A and Fig. 10B show that an IAV (76.98°C) melting peak and an RSV (70.84°C) melting peak appear in a PCR reaction well with addition of an IAV positive template and a RSV positive template in a CY5 channel, respectively.

Fig. 11 shows that dual melting peaks of IAV (76.98°C) and RSV (70.84°C) simultaneously appear in a PCR reaction well with addition of both an IAV positive template and a RSV positive template in a CY5 channel.

It may be seen from Figs. 6-11 that, when the primer probe design of the present invention was used, a multiplex detection may be achieved in a single tube reaction, and two-dimensional analysis of a fluorescence channel and melting temperature in a single tube reaction may be simultaneously performed, that is, by using a same fluorescence channel, different targets may be detected by means of melting temperature characteristics; and by using different fluorescent channels, the detection of target species may be achieved by multiplying the number of the fluorescent channels by the melting temperature characteristic.

### Comparative Example 1: Impact of a primer dimer on detection results in different primer probe design methods (detection target: respiratory syncytial virus (RSV))

(1) Base sequences of the primer probes used for detecting respiratory syncytial virus (RSV) are shown in Table 10.

**Table 10: Base sequences of primer probe**

| Name of primer probe | Number | Nucleotide sequence (5'-3') | Label |
|---|---|---|---|
| P1-1 | SEQ ID NO:1 | CCCTGGGAGCTCATGGAGGTACTG GTCCTGTCGACGCTGCGCACACTG GGACGGCACCG | T14VIC, T26BHQ1 |
| | | | 3' Spacer C3 |
| P2-5 | SEQ ID NO:25 | AGCGATGGTGTCGAGAGGGT | 3'BHQ1 |
| | | | 5' VIC |
| R1-1 | SEQ ID NO:2 | CGGCACCGAAATATTGCAGATGTG ACACCTTAGTA | |
| F1-1 | SEQ ID NO:5 | GCTCCCAGGGTTTAACATTAAGCA AGAAGAGGAAAMGAAGAT | |
| R2-5 | SEQ ID NO:26 | AGCGATGGTGTCGAGAGGGTAAAT ATTGCAGATGTGACACCTTAGTA | / |
| F2-5 | SEQ ID NO:27 | AACATTAAGCAAGAAGAGGAAAM GAAGAT | / |
| F3-5 | SEQ ID NO:28 | TTTTTTTTTTACTAAGGTGTCACAT CTGCAATA | / |

In the above primer probe:
the forward primer F1-1 (SEQ ID NO: 5) is a specific primer designed for a target sequence of respiratory syncytial virus, and the F1-1 has a total length of 42 bp, wherein a 1^{st} to 29^{th} bases at 3' end thereof serve as a target sequence binding region 1, and a 1^{st} to 10^{th} bases at 5' end thereof serve as a probe anchoring region (A), which is reversely complementary with a sequence of a 1^{st} to 10^{th} bases (i.e. a primer anchoring region (A')) at 5' end of the probe P1-1 (SEQ ID NO: 1);
the reverse primer R1-1 (SEQ ID NO: 2) is a specific primer designed for the target sequence of respiratory syncytial virus, and the R1-1 has a total length of 35 bp, wherein a 1^{st} to 24^{th} bases at 3' end thereof serve as a target sequence binding region 2, and a sequence of a 1^{st} to 8^{th} bases at 5' end thereof is completely identical to a sequence of a 1^{st} to 8^{th} bases at 3' end of the probe P1-1 (SEQ ID NO: 1);
the forward primer F2-5 (SEQ ID NO: 27) is a specific primer designed for the target sequence of respiratory syncytial virus, and the F2-5 has a total length of 29 bp, and is identical to a sequence of a 1^{st} to 29^{th} bases at 3' end of the forward primer F1 (SEQ ID NO: 5);
the reverse primer R2-5 (SEQ ID NO: 26) is a specific primer designed for the target sequence of respiratory syncytial virus, and the R2-5 has a total length of 47 bp, wherein a 1^{st} to 24^{th} bases at 3' end thereof serve as a target sequence binding region which is identical to a sequence of a 1^{st} to 24^{th} bases at 3' end of R1, and a sequence of a 1^{st} to 20^{th} bases at 5' end thereof is completely identical to a base sequence of the probe P2 (SEQ ID NO: 25);
the forward primer F3-5 (SEQ ID NO: 28) is a specific dimeric primer designed for the target sequence of respiratory syncytial virus, and the F3-5 has a total length of 30 bp, wherein a 1^{st} to 21^{st} bases at 3' end thereof are reversely complementary with a sequence of a 1^{st} to 21^{st} bases at the 3'end of the reverse primer R1 (SEQ ID NO: 2); and
the probe P2-5 (SEQ ID NO: 25) is a self-designed sequence that is not complementary with any part of the target sequence, and the P2-5 has a total length of 20 bp, is identical to a sequence of a 1^{st} to 20^{th} bases at 5' end of R2. A first detection group and a second detection group were at two ends of the probe P2 (SEQ ID NO: 25), respectively.

When the primers F1-1, R1-1, and the P1-1 in Table 10 are used to specifically amplify the target sequence of respiratory syncytial virus, the forward primer F1-1 (SEQ ID NO: 5) and the reverse primer R1-1 (SEQ ID NO: 2) amplify the nucleic acid of respiratory syncytial virus to obtain a double-stranded pre-amplification product. The probe signal detection region (H) of the probe P1-1 (SEQ ID NO: 1) is complementarily paired with the reverse-complementary sequence (h') of the primer signal detection region at the 3' end of one single-stranded pre-amplification product (S1), and the primer anchoring region (A') of the probe P1-1 is complementarily paired with the probe anchoring region (A) at 5' end of the single-stranded pre-amplification product (S1), and the 3' end of the one single-stranded pre-amplification product (S1) may continue to extend to 5' end of probe P1-1, to obtain a reverse-complementary sequence of the probe P1-1 (SEQ ID NO: 1), so as to obtain a secondary amplification double-stranded product formed with the probe P1-1 (SEQ ID NO: 1). At this time, a distance between the first detection group and the second detection group is increased due to a change from single strand to double strand, and the efficiency of fluorescence resonance energy transfer is decreased, such that a fluorescence signal change is produced and may be detected by an instrument. After the completion of PCR amplification, a melting curve analysis is performed, and the secondary amplification double-stranded product formed with the probe P1-1 will melt at a certain melting temperature, at this time, the probe P1-1 with the the first detection group and the second detection group will become a single-stranded state, such that a fluorescence signal change is produced again and may be detected by the instrument.

When the primers F2-5, R2-5, and the probe P2-5 in Table 10 are used to specifically amplify the target sequence of respiratory syncytial virus, the F2-5 (SEQ ID NO: 27) and the R2-5 (SEQ ID NO: 26) amplify the nucleic acid of respiratory syncytial virus to obtain a double-stranded pre-amplification product, wherein a 5' end of one single-stranded pre-amplification product is reversely complementary with the probe P2 (SEQ ID NO: 25). At this time, a distance between the first detection group and the second detection group at two ends of the probe P2-5 is increased due to a change from single strand to double strand, and the efficiency of fluorescence resonance energy transfer is decreased, such that a fluorescence signal change is produced and may be detected by an instrument. After the completion of PCR amplification, a melting curve analysis is performed, and the secondary amplification double-stranded product formed with the probe P2-5 will melt at a certain melting temperature, at this time, the probe P2-5 with the the first detection group and the second detection group will become a single-stranded state, such that a fluorescence signal change is produced again and may be detected by the instrument.

When there is no respiratory syncytial virus in the sample to be detected, an amplification detection is performed by using the primers F3-5, R1-1, and the probe P1-1 in Table 10. A double-stranded primer dimer is obtained by reverse-complementary amplification of the forward primer F3-5 (SEQ ID NO: 28) and the reverse primer R1-1 (SEQ ID NO: 2), and since the forward primer F3-5 does not contain a probe anchoring region (A) at 5' end thereof, neither of the single strands of the obtained double-stranded primer dimer contains the probe anchoring region, such that it is impossible to be subjected to a secondary amplification. After amplification, a melting curve analysis is performed, and no signal was generated due to the absence of a secondary amplification double-stranded product formed with theprobe P1-1 (SEQ ID NO: 1).

When there is no respiratory syncytial virus in the sample to be detected, an amplification detection is performed by using the primers F3-5, R2-5, and the probe P2-5 in Table 10. A double-stranded primer dimer is obtained by reverse-complementary amplification of the forward primer F3-5 (SEQ ID NO: 28) and the reverse primer R2-5 (SEQ ID NO: 26), wherein one single-stranded product may be reversely complementary with the probe P2-5 (SEQ ID NO: 25), such that a distance between the first detection group and the second detection group at two ends of the probe P2-5 is increased due to a change from single strand to double strand, and the efficiency of fluorescence resonance energy transfer is decreased, and thus a fluorescence signal change is produced and may be detected by an instrument, thereby generating a false positive signal. After the completion of PCR amplification, a melting curve analysis is performed, and the double-stranded product formed with the probe P2-5 will melt at a certain melting temperature, at this time, the probe P2-5 with the the first detection group and the second detection group will become a single-stranded state, such that a fluorescence signal change is produced and may be detected by the instrument, thereby generating a false positive signal.

(2) A detection of respiratory syncytial virus (RSV) was performed by using the above primer probe.

Reaction systems used for detection were shown in Table Table 11.

**Table 11: Singleplex PCR detection reaction system for respiratory syncytial virus**

| Reagents and components | | Concentration |
|---|---|---|
| Singleplex detection reaction system for | 2× PCR Reaction Buffer | 1× |
| | DNA Polymerase | 2U |
| | Reverse transcriptase | 5U |
| | Forward primer (F1-1) | 500 nM |
| respiratory syncytial virus (P1-1 probe system) System 1 | Reverse primer (R1 -1) | 100 nM |
| | Probe (P1-1) | 400 nM |
| | Nucleic acid template | 30 copies |
| | Ultra pure water | Adding to 20 µL |
| Singleplex detection reaction system for respiratory syncytial virus (P1-1 probe primer dimer system) System 2 | 2× PCR Reaction Buffer | 1× |
| | DNA Polymerase | 2U |
| | Reverse transcriptase | 5U |
| | Forward primer (F3-5) | 100 nM |
| | Reverse primer (R1-1) | 100 nM |
| | Probe (P1-1) | 400 nM |
| | Nucleic acid template | 30 copies |
| | Ultra pure water | Adding to 20 µL |
| Singleplex detection reaction system for respiratory syncytial virus (P2-5 probe system) System 3 | 2× PCR Reaction Buffer | 1× |
| | DNA Polymerase | 2U |
| | Reverse transcriptase | 5U |
| | Forward primer (F2-5) | 500 nM |
| | Reverse primer (R2-5) | 100 nM |
| | Probe (P2-5) | 400 nM |
| | Nucleic acid template | 30 copies |
| | Ultra pure water | Adding to 20 µL |
| Singleplex detection | 2× PCR Reaction Buffer | 1× |
| | DNA Polymerase | 2U |
| reaction system for respiratory syncytial virus (P2-5 probe primer dimer system) System 4 | Reverse transcriptase | 5U |
| | Forward primer (F3-5) | 100 nM |
| | Reverse primer (R2-5) | 100 nM |
| | Probe (P2-5) | 400 nM |
| | Nucleic acid template | 30 copies |
| | Ultra pure water | Adding to 20 µL |

| | | |
|---|---|---|
| Note: The 2xPCR reaction buffer comprises 3 mM MgCl₂, 30 mM Tris-HCl buffer with a pH of 8.3, 0.5 mM dNTP, and 70 mM (NH₄)₂SO₄. | | |

Nucleic acid template: an in-vitro transcribed RNA of the target sequence of respiratory syncytial virus was used as the nucleic acid template. The ultra-pure water was used as a no template control (NTC).

### Reaction preparation:

After the preparation of the nucleic acid template was completed, 4 reaction systems were prepared according to the ratios described in Table 11.

### PCR amplification and melting curve analysis:

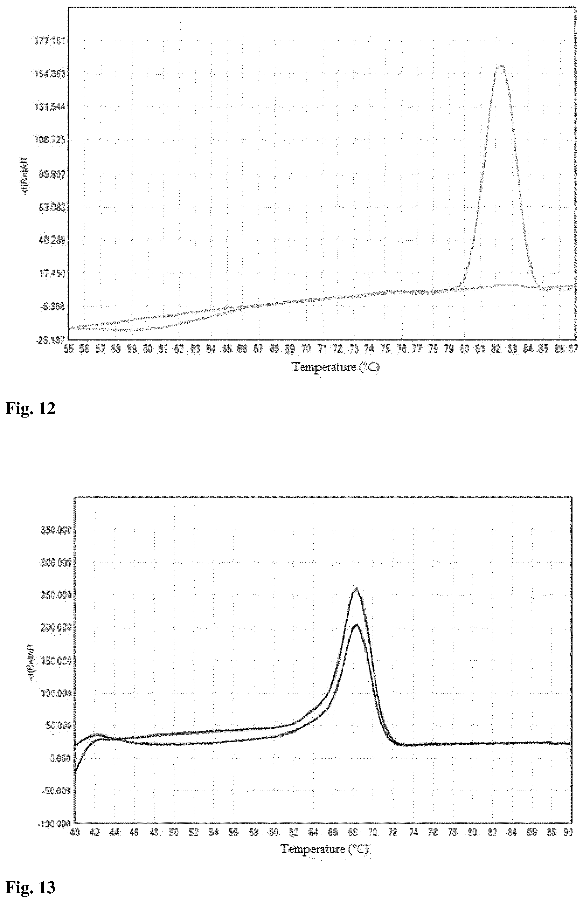

After a PCR tube was sealed by a cap, the sample was gently and uniformly mixed, and centrifuged for a short time and then left for 5 min at a room temperature. The PCR tube was put in a handheld centrifuge again, centrifuged for a short time, and then transferred to a tray of SLAN real-time fluorescence quantitative PCR instrument from Shanghai Hongshi Medical Technology Co., Ltd. The procedure used was as follows: performing reverse transcription at 55 °C for 15 minutes; performing pre-denaturation at 95°C for 3 minutes; performing denaturation at 94 °C for 10 seconds, and performing annealing and extension at 56 °C for 30 seconds, for a total of 45 cycles, without lighting; performing denaturation at 94 °C for 150 seconds, and perfroming incubation at a constant temperature of 40°C for 300 seconds; and performing melting curve analysis at 40-85°C, with a heating rate of 0.05°C/s and with lighting, to obtain melting curves, as shown in Figs. 12-13.

### Data analysis:

The results were interpreted via Tm values of the melting curves by using an analysis software of the SLAN real-time fluorescence quantitative PCR instrument from Shanghai Hongshi Medical Technology Co., Ltd.

Fig. 12 is a composite view of the results for system 1 and system 2 in Table 11, which shows that a melting peak with a Tm of 82.23°C is formed by using the probe in a PCR reaction well with addition of a RSV positive template in a VIC channel, while the primer dimer does not produce a melting peak; and

Fig. 13 is a composite view of the results for system 3 and system 4 in Table 11, which shows that a melting peak with a Tm of 68.48°C and a primer dimer melting peak with a Tm value of 68.52°C are formed by using the probe P2 in an PCR reaction well with addition of a RSV positive template in a FAM channel.

It may be seen from Fig. 12 that: in the system 1, the primer probe design method of the present invention was adopted, and in the case that the probe anchoring region of the single-stranded pre-amplification product was complementary with the primer anchoring region of the probe, a reverse-complementary sequence of the primer signal detection region thereof may be reversely complementary with the probe signal detection region of the probe, so as to achieve that the 3' of the reverse-complementary sequence of the primer signal detection region continues to extend to the 5' of the probe, thereby completing a secondary amplification in which the single-stranded pre-amplification product was used as a primer and the probe was used as a template, and obtaining a double-stranded amplification product which was partially or completely complementary with the probe, and when the melting curve analysis was performed, a clear melting peak with a Tm value of 82.23°C was obtained. In system 2, only the reverse primer was completely complemented, to obtain a pre-amplification product comprising, from 5' to 3', a reversely complementary sequence of the reverse primer and a reversely complementary sequence of the primer signal detection region, and since the pre-amplification product does not contain a probe anchoring region, it cannot be subjected to annealing and extension with the probe under the set annealing conditions, such that a signal will not be produced. Thus it may be seen that, when the primer probe design method of the present invention was adopted, an influence of a primer dimer on the detection results will be effectively avoided.

It may be seen from Fig. 13 that, the products formed by systems 3 and 4 may be completely complementary with the probe P2, thereby generating signals. Thus it can be seen that, when a traditional primer probe design method was adopted, the generated primer dimer was prone to cause a false positive, thereby affecting the accuracy of detection results.

### Example 5: Singleplex PCR results of a forward primer without a primer anchoring region:

Singleplex PCR detection for non-small cell lung cancer EGFR exon 20 H773_V774insH mutation (1) Base sequences of a primer probe used for Singleplex PCR detection for non-small cell lung cancer EGFR exon 20 H773_V774insH mutation are shown in Table 12.

**Table 12: Base sequences of primer probe**

| Name of primer probe | Number | Nucleotide sequence (5'-3') | Label |
|---|---|---|---|
| P1-1 | SEQ ID NO:1 | CCCTGGGAGCTCATGGAATCGCTG GTCCTGTCGACGCTGCGCACACTG GGACGGCACCG | T14-CY5, T26BHQ1 |
| | | | 3' Spacer C3 |
| R1-6 | SEQ ID NO:29 | CACTGGGACGGCACCGAAAAGTCT GAACGACCTACACTCC | / |
| F1-6 | SEQ ID NO:30 | CCCAGCAGCTGACACA | / |

In the above primer probe:
the forward primer F1-6 (SEQ ID NO: 30) is a specific primer designed for a target sequence of non-small cell lung cancer EGFR exon 20 H773_V774insH mutation, and the F1-6 has a total length of 16 bp, wherein a 1^{st} to 16^{th} bases at 3' end thereof serve as a target sequence binding region 1; and
the reverse primer R1-6 (SEQ ID NO: 29) is a specific primer designed for the target sequence of non-small cell lung cancer EGFR exon 20 H773_V774insH mutation, and the R1-6 has a total length of 40 bp, wherein a 1^{st} to 24^{th} bases at 3' end thereof serve as a target sequence binding region 2, and a 1^{st} to 16^{th} bases at 5' end thereof serve as a primer signal detection region (h), which is identical to a sequence of a 1^{st} to 16^{th} bases at 3' end of the probe P1-1 (SEQ ID NO: 1).

(2) A singleplex PCR detection for non-small cell lung cancer EGFR exon 20 H773_V774insH mutation was performed by using the above primer probe.

A reaction system used for detection was shown in Table 13.

**Table 13: Singleplex PCR detection reaction system for non-small cell lung cancer EGFR exon 20 H773_V774insH mutation**

| Reagents and components | Concentration |
|---|---|
| 2 x PCR reaction buffer | 1× |
| DNA polymerase | 2U |
| Forward primer (F1-6) | 500 nM |
| Reverse primer (R1-6) | 100 nM |
| Probe (P1-1) | 400 nM |
| Nucleic acid template | 30 copies |
| Ultra pure water | Adding to 20 µL |

| | |
|---|---|
| Note: The 2xPCR reaction buffer comprises 3 mM MgCl₂, 30 mM Tris-HCl buffer with a pH of 8.3, 0.5 mM dNTP, and 70 mM (NH₄)₂SO₄. | |

Nucleic acid template: a positive sample of the target sequence of non-small cell lung cancer EGFR exon 20 H773_V774insH mutation was used as the nucleic acid template, and the reverse primer (R) with a signal detection region sequence was used to separately detect the target sequence of non-small cell lung cancer EGFR exon 20 H773_V774insH mutation, to verify a detection ability for single mutation. The ultra-pure water was used as a no template control (NTC).

### Reaction preparation:

After the preparation of the nucleic acid template was completed, the reaction system was prepared according to the ratios described in Table 13.

### PCR amplification and melting curve analysis:

After a PCR tube was sealed by a cap, the sample was gently and uniformly mixed, and centrifuged for a short time and then left for 5 min at a room temperature. The PCR tube was put in a handheld centrifuge again, centrifuged for a short time, and then transferred to a tray of SLAN real-time fluorescence quantitative PCR instrument from Shanghai Hongshi Medical Technology Co., Ltd. The procedure used was as follows: performing pre-denaturation at 95°C for 3 minutes; performing denaturation at 94 °C for 10 seconds, and performing annealing and extension at 56 °C for 30 seconds, for a total of 45 cycles, without lighting; performing denaturation at 94 °C for 150 seconds, and perfroming incubation at a constant temperature of 40°C for 300 seconds; and performing melting curve analysis at 40-85°C, with a heating rate of 0.05°C/s and with lighting, to obtain a melting curve.

### Data analysis:

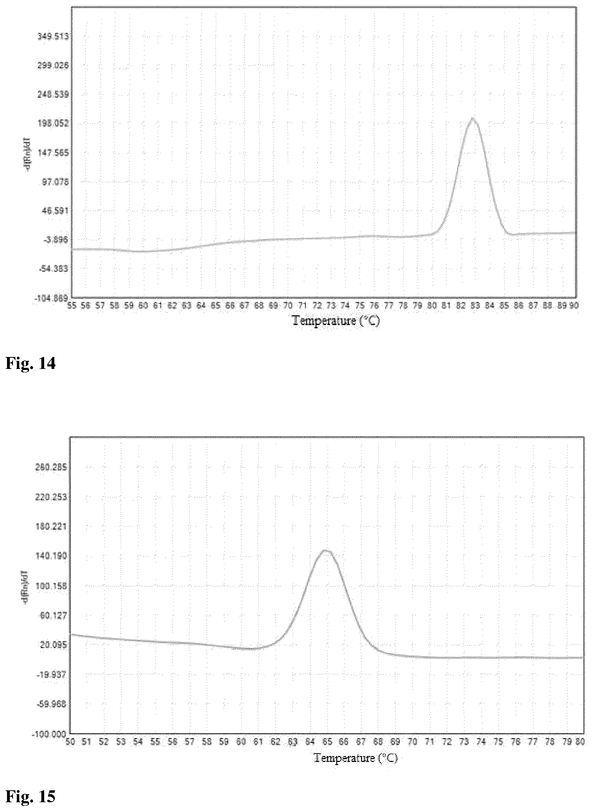

The results were interpreted via a Tm value of the melting curve by using an analysis software of the SLAN real-time fluorescence quantitative PCR instrument from Shanghai Hongshi Medical Technology Co., Ltd. Fig. 14 is a melting curve of a singleplex PCR non-small cell lung cancer EGFR exon 20 H773_V774insH mutation positive sample amplified by the reverse primers (R) with a signal detection region.

It may be seen from Fig. 14 that, when the primer probe of the present invention was used, the non-small cell lung cancer EGFR exon 20 H773_V774insH mutation may be detected.

### Example 6: Singleplex PCR results of omega form with an anchoring region overlapped with the detection group: Singleplex PCR detection for non-small cell lung cancer EGFR exon 20 H773_V774insH mutation

(1) Base sequences of a primer probe used for Singleplex PCR detection for non-small cell lung cancer EGFR exon 20 H773_V774insH mutation are shown in Table 14.

**Table 14: Base sequences of primer probe**

| Name of primer probe | Number | Nucleotide sequence (5'-3') | Label |
|---|---|---|---|
| P1-1 | SEQ ID NO:1 | CCCTGGGAGCTCATGGAATCGCTG GTCCTGTCGACGCTGCGCACACTG GGACGGCACCG | T14-CY5, T26BHQ1 3' Spacer C3 |
| R1-7 | SEQ ID NO:31 | GCTCATGGAAAAGTCTGAACGACC TACACTCC | / |
| F1-7 | SEQ ID NO:32 | GTCGACAGGACCATTTCCCAGCAG CTGACACA | / |

In the above primer probe:
the forward primer F1-7 (SEQ ID NO: 32) is a specific primer designed for a target sequence of non-small cell lung cancer EGFR exon 20 H773_V774insH mutation, and the F1-7 has a total length of 32 bp, wherein a 1^{st} to 16^{th} bases at 3' end thereof serve as a target sequence binding region; and a 1^{st} to 13^{rd} bases at 5' end thereof serve as a probe anchoring region (A), which is reversely complementary with a sequence of a 23^{rd} to 35^{th} bases (i.e. a primer anchoring region (A')) at 5' end of the probe P1-1 (SEQ ID NO: 1); and
the reverse primer R1-7 (SEQ ID NO: 31) is a specific primer designed for the target sequence of non-small cell lung cancer EGFR exon 20 H773_V774insH mutation, and the R1-7 has a total length of 32 bp, wherein a 1^{st} to 24^{th} bases at 3' end thereof serve as a target sequence binding region, and a 1^{st} to 8^{th} bases at 5' end thereof serve as a primer signal detection region (h2), which is identical to a sequence of a 44^{th} to 51^{st} bases at 3' end of the probe P1-1 (SEQ ID NO: 1).

(2) A singleplex PCR detection for non-small cell lung cancer EGFR exon 20 H773_V774insH mutation was performed by using the above primer probe.

A reaction system used for detection was shown in Table 15.

**Table 15: Singleplex PCR detection reaction system for non-small cell lung cancer EGFR exon 20 H773_V774insH mutation**

| Reagents and components | Concentration |
|---|---|
| 2 x PCR reaction buffer | 1× |
| DNA polymerase | 2U |
| Forward primer (F1-7) | 500 nM |
| Reverse primer (R1-7) | 100 nM |
| Probe (P1-1) | 400 nM |
| Nucleic acid template | 30 copies |
| Ultra pure water | Adding to 20 µL |

| | |
|---|---|
| Note: The 2xPCR reaction buffer comprises 3 mM MgCl₂, 30 mM Tris-HCl buffer with a pH of 8.3, 0.5 mM dNTP, and 70 mM (NH₄)₂SO₄. | |

Nucleic acid template: a positive sample of the target sequence of non-small cell lung cancer EGFR exon 20 H773_V774insH mutation was used as the nucleic acid template, and the reverse primer (R) with a signal detection region sequence was used to separately detect the target sequence of non-small cell lung cancer EGFR exon 20 H773_V774insH mutation, to verify a detection ability for single mutation. The ultra-pure water was used as a no template control (NTC).

### Reaction preparation:

After the preparation of the nucleic acid template was completed, the reaction system was prepared according to the ratios described in Table 15.

### PCR amplification and melting curve analysis:

After a PCR tube was sealed by a cap, the sample was gently and uniformly mixed, and centrifuged for a short time and then left for 5 min at a room temperature. The PCR tube was put in a handheld centrifuge again, centrifuged for a short time, and then transferred to a tray of SLAN real-time fluorescence quantitative PCR instrument from Shanghai Hongshi Medical Technology Co., Ltd. The procedure used was as follows: performing pre-denaturation at 95°C for 3 minutes; performing denaturation at 94 °C for 10 seconds, and performing annealing and extension at 56 °C for 30 seconds, for a total of 45 cycles, without lighting; performing denaturation at 94 °C for 150 seconds, and perfroming incubation at a constant temperature of 40°C for 300 seconds; and performing melting curve analysis at 40-85°C, with a heating rate of 0.05°C/s and with lighting, to obtain a melting curve.

### Data analysis:

The results were interpreted via a Tm value of the melting curve by using an analysis software of the SLAN real-time fluorescence quantitative PCR instrument from Shanghai Hongshi Medical Technology Co., Ltd. Fig. 15 is a melting curve of a singleplex PCR non-small cell lung cancer EGFR exon 20 H773_V774insH mutation positive samples amplified by a reverse primers (R) with a signal detection region.

It may be seen from Fig. 15 that, when the primer probe of the present invention was used, the non-small cell lung cancer EGFR exon 20 H773_V774insH mutation may be detected.

### Example 7: Multiple PCR detection for IMP mutation in genes resistant to carbapenems, OXA-48 mutation in genes resistant to carbapenems, KPC mutation in genes resistant to carbapenems, and VIM mutation in genes resistant to carbapenems

(1) Base sequence of a primer probe used for multiplex PCR detection for IMP mutation in genes resistant to carbapenems, OXA-48 mutation in genes resistant to carbapenems, KPC mutation in genes resistant to carbapenems, and VIM mutation in genes resistant to carbapenems are shown in Table 16.

**Table 16 Base sequences of primer probe in Example 7**

| Name of primer probe | Number | Nucleotide sequence (5'-3') | Label |
|---|---|---|---|
| P7-1 | SEQ ID NO:33 | GTGCACACGGTACTGTCAGCTGA ACTGGTCTCACTGCAGCGCTGTC CGAGCTCGACGTG | T14-FAM, T26-BHQ2 3'Spacer C3 |
| P7-2 | SEQ ID NO:34 | GGCTTCGAGCACTTGCACTGATC CGATCGACGTGCGACCATGACGG AGAGCAGGACGAG | T14-ROX T27-BHQ2 3' Spacer C3 |
| F7-1 | SEQ ID NO:35 | CCGTGTGCACAAAAAACTGACAC AGGGCTCTGCA | / |
| R7-1 | SEQ ID NO:36 | GCTCGACGTGTTTGTCATTTGCCG TGCCATACACT | / |
| F7-2 | SEQ ID NO:37 | CCGTGTGCACAAATAGTAGTATGG CTGCCTGATAGGA | / |
| R7-2 | SEQ ID NO:38 | TGCAGCGCTTTGTCACCCAAATA ACCTAGACCGT | / |
| F7-3 | SEQ ID NO:39 | CCGTGTGCACAAAATTGGCGAGG CACGTATGA | / |
| R7-3 | SEQ ID NO:40 | GCTGAACTGGTTTTCACCGTCGA GCCAGAAACTGTCTA | / |
| F7-4 | SEQ ID NO:41 | GCTCGAAGCCAAAAGCAAATTGG ACTTCCCGTAACG | / |
| R7-4 | SEQ ID NO:42 | TGCGACCATGATTTCCTTCTAGAG AATGCGTGGGAATCT | / |

In the above primer probe:
Both of the forward primer F7-1 (SEQ ID NO: 35) and the reverse primer R7-1 (SEQ ID NO: 36) are specific primers designed for a target sequence of KPC mutation in genes resistant to carbapenems. The F7-1 has a total length of 34 bp, wherein a 1^{st} to 21^{st} bases at 3' end thereof serve as a target sequence binding region 1, and a 1^{st} to 10^{th} bases at 5' end thereof serve as a probe anchoring region (A1), which is reversely complementary with a sequence of a 1^{st} to 10^{th} bases (i.e. a primer anchoring region (A1')) of the probe P7-1 (SEQ ID NO: 33). The R1 has a total length of 35 bp, wherein a 1^{st} to 22^{nd} bases at 3' end thereof serve as a target sequence binding region 2, and a 1^{st} to 10^{th} bases at 5' end thereof serve as a primer signal detection region (h1), which is identical to a sequence of a 1^{st} to 10^{th} bases at 3' end of the probe P7-1 (SEQ ID NO: 33).
Both of the forward primer F7-2 (SEQ ID NO: 37) and the reverse primer R7-2 (SEQ ID NO: 38) are specific primers designed for a target sequence of IMP mutation in genes resistant to carbapenems. The F7-2 has a total length of 37 bp, wherein a 1^{st} to 24^{th} bases at 3' end thereof serve as a target sequence binding region 3, and a 1^{st} to 10^{th} bases at 5' end thereof serve as a probe anchoring region (A1), which is reversely complementary with a sequence of a 1^{st} to 10^{th} bases (i.e. a primer anchoring region (A1')) at 5' end of the probe P7-1 (SEQ ID NO: 33). The R2 has a total length of 37 bp, wherein a 1^{st} to 23^{rd} bases at 3' end thereof serve as a target sequence binding region 4, and a 1^{st} to 8^{th} bases at 5' end thereof serve as a primer signal detection region (h2), which is identical to a sequence of a 18^{th} to 25^{th} bases at 3' end of the probe P7-1 (SEQ ID NO: 33).
Both of the forward primer F7-3 (SEQ ID NO: 39) and the reverse primer R7-3 (SEQ ID NO: 40) are specific primers designed for a target sequence of OXA-48 mutation in genes resistant to carbapenems. The F3 has a total length of 32 bp, wherein a 1^{st} to 19^{th} bases at 3' end thereof serve as a target sequence binding region 5, and a 1^{st} to 10^{th} bases at 5' end thereof serve as a probe anchoring region (A1), which is reversely complementary with a sequence of a 1^{st} to 10^{th} bases (i.e. a primer anchoring region (A1')) at 5' endof the probe P7-1 (SEQ ID NO: 33). The R3 has a total length of 35 bp, wherein a 1^{st} to 24^{th} bases at 3' end thereof serve as a target sequence binding region 6, and a 1^{st} to 11^{th} bases at 5' end thereof serve as a primer signal detection region (h3), which is identical to a sequence of a 31^{st} to 41^{st} bases at 3' end of the probe P7-1 (SEQ ID NO: 1). Both of the forward primer F7-4 (SEQ ID NO: 41) and the reverse primer R7-4 (SEQ ID NO: 42) are specific primers designed for a target sequence of VIM mutation in genes resistant to carbapenems. The F7-4 has a total length of 36 bp, wherein a 1^{st} to 23^{rd} bases at 3' end thereof serve as a target sequence binding region 7, and a 1^{st} to 10^{th} bases at 5' end thereof serve as a probe anchoring region (A2), which is reversely complementary with a sequence of a 1^{st} to 10^{th} bases (i.e. a primer anchoring region (A2')) at 5' end of the probe P7-2 (SEQ ID NO: 34). The R7-4 has a total length of 39 bp, wherein a 1^{st} to 25^{th} bases at 3' end thereof serve as a target sequence binding region 8, and a 1^{st} to 10^{th} bases at 5' end thereof serve as a primer signal detection region (h4), which is identical to a sequence of a 17^{th} to 27^{th} bases at 3' end of the probe P7-2 (SEQ ID NO: 34).

The characteristic Tm values of the above probes are shown in Table 17:

**Table 17**

| Fluorescent channel | Melting temperature (Tm, °C) | | |
|---|---|---|---|
| | 69≤Tm<74 | 75≤Tm<80 | 80≤Tm<85 |
| ROX | / | VIM mutation | / |
| FAM | OXA-48 mutation | IMP mutation | KPC mutation |

(2) A multiplex PCR detection for IMP mutation in genes resistant to carbapenems, OXA-48 mutation in genes resistant to carbapenems, KPC mutation in genes resistant to carbapenems, and VIM mutation in genes resistant to carbapenems was performed by using the above primer probe. A reaction systems used for detection was shown in Table 18.

**Table 18: Multiple PCR detection reaction system for multiplex PCR detection for KPC mutation in genes resistant to carbapenems, IMP mutation in genes resistant to carbapenems, OXA-48 mutation in genes resistant to carbapenems, and VIM mutation in genes resistant to carbapenems**

| Reagents and components | Concentration |
|---|---|
| 2× PCR Reaction Buffer | 1× |
| DNA Polymerase | 2U |
| Probe (P7-1) | 400 nM |
| Forward primer (F7-1) | 500 nM |
| Reverse primer (R7-1) | 100 nM |
| Forward primer (F7-2) | 500 nM |
| Reverse primer (R7-2) | 100 nM |
| Forward primer (F7-3) | 500 nM |
| Reverse primer (R7-3) | 100 nM |
| Probe (P7-2) | 400 nM |
| Forward primer (F7-4) | 500 nM |
| Reverse primer (R7-4) | 100 nM |
| A sample of KPC mutation in genes resistant to carbapenems, a sample of IMP mutation in genes resistant to carbapenems, a sample of OXA-48 mutation in genes resistant to carbapenems, or a sample of VIM mutation in genes resistant to carbapenems | 500 copies |
| Ultra pure water | Adding to 20 µL |

| | |
|---|---|
| Note: The 2xPCR reaction buffer comprises 3 mM MgCl₂, 30 mM Tris-HCl buffer with a pH of 8.3, 0.5 mM dNTP, and 70 mM (NH₄)₂SO₄. | |

Nucleic acid template: a sample of KPC mutation in genes resistant to carbapenems, a sample of IMP mutation in genes resistant to carbapenems, a sample of OXA-48 mutation in genes resistant to carbapenems, and a sample of VIM mutation in genes resistant to carbapenems were uses as nucleic acid templates respectively, to perform a detection for single gene mutation in a multiplex system. The ultra-pure water was used as a no template control (NTC).

### Reaction preparation:

After the preparation of the nucleic acid templates were completed, the reaction system was prepared according to the ratios described in Table 18.

### PCR amplification and melting curve analysis:

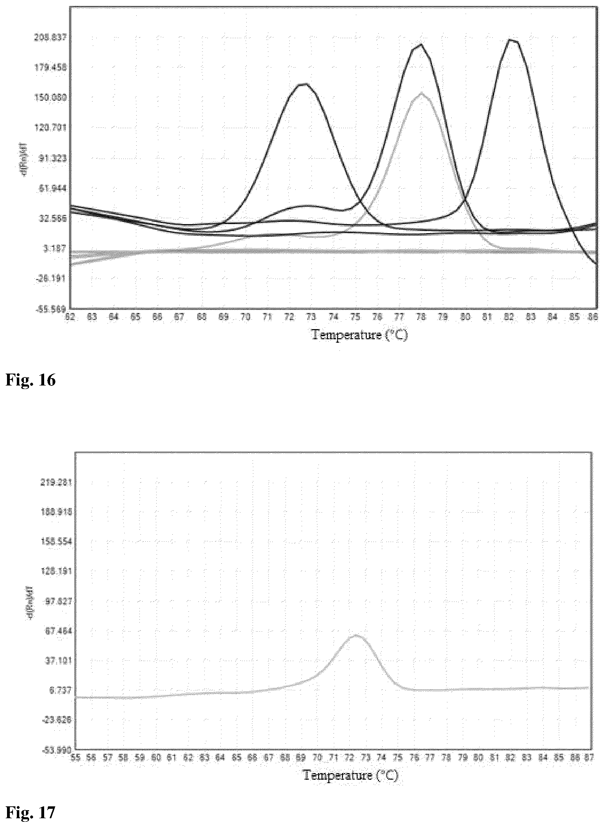

After a PCR tube was sealed by a cap, the sample was gently and uniformly mixed, and centrifuged for a short time and then left for 5 min at a room temperature. The PCR tube was put in a handheld centrifuge again, centrifuged for a short time, and then transferred to a tray of SLAN real-time fluorescence quantitative PCR instrument from Shanghai Hongshi Medical Technology Co., Ltd. The procedure used was as follows: performing pre-denaturation at 95°C for 3 minutes; performing denaturation at 94 °C for 10 seconds, and performing annealing and extension at 56 °C for 30 seconds, for a total of 45 cycles, without lighting; performing denaturation at 94 °C for 150 seconds, and perfroming incubation at a constant temperature of 40°C for 300 seconds; and performing melting curve analysis at 62-86°C, with a heating rate of 0.05°C/s and with lighting, to obtain melting curves, as shown in Fig. 16 respectively.

### Data analysis:

The results were interpreted via Tm values of the melting curves by using an analysis software of the SLAN real-time fluorescence quantitative PCR instrument from Shanghai Hongshi Medical Technology Co., Ltd.

Fig. 16 shows that, with the addition of the sample of KPC mutation in genes resistant to carbapenems, the sample of IMP mutation in genes resistant to carbapenems, the sample of OXA-48 mutation in genes resistant to carbapenems, and the sample of VIM mutation in genes resistant to carbapenems in PCR reaction wells, melting peaks of KPC mutation (82.26°C), IMP mutation (77.88°C), and OXA-48 mutation (72.62°C) appeare in a FAM channel, and a melting peak of VIM mutation (78.02°C) appeares in a ROX channel, respectively.

### Example 8: Singleplex PCR detection for non-small cell lung cancer EGFR exon 21 L861Q mutation

(1) Base sequences of a primer probe used for Singleplex PCR detection for non-small cell lung cancer EGFR exon 21 L861Q mutation are shown in Table 19.

**Table 19: Base sequences of primer probe in Example 8**

| Name of primer probe | Number | Nucleotide sequence (5'-3') | Label |
|---|---|---|---|
| P8-1 | SEQ ID NO:43 | AGACGCACGGTGACATGTGTTTGA CTCAGTTCTGCTGCTGCCCACCGTC TGGAGTGCGT | T16-HEX, T31-BHQ1 3' Spacer C3 |
| R8-1 | SEQ ID NO:44 | CTGCTGCTGCCCAAAGCCTCCTTCT GCATGGTATTCT | / |
| F8-1 | SEQ ID NO:45 | AGATTTTGGGCTGGCCAAACA | / |

In the above primer probe:
the forward primer F8-1 (SEQ ID NO: 45) is a specific primer designed for a target sequence of non-small cell lung cancer EGFR exon 21 L861Q mutation, and the F8-1 has a total length of 21 bp, wherein a 1^{st} to 21^{st} bases at 3' end thereof serve as a target sequence binding region; and
the reverse primer R8-1 (SEQ ID NO: 44) is a specific primer designed for the target sequence of non-small cell lung cancer EGFR exon 21 L861Q mutation, and the R8-1 has a total length of 37 bp, wherein a 1^{st} to 22^{nd} bases at 3' end thereof serve as a target sequence binding region, and a 1^{st} to 12t^{h} bases at 5' end thereof serve as a primer signal detection region (h1), which is identical to a sequence of a 17^{th} to 28^{th} bases at 3' end of the probe P8-1 (SEQ ID NO: 43).

(2) A PCR detection was performed by using the above primer probe, and a reaction system used for detection was shown in Table 20.

**Table 20 Reaction system for detection in Example 8**

| Reagents and components | Concentration |
|---|---|
| 2× PCR Reaction Buffer | 1× |
| DNA Polymerase | 2U |
| Forward primer (F8-1) | 500 nM |
| Reverse primer (R8-1) | 100 nM |
| Probe (P) | 400 nM |
| Nucleic acid template | 300 copies |
| Ultra pure water | Adding to 20 µL |

| | |
|---|---|
| Note: The 2XPCR reaction buffer comprises 3 mM MgCl₂, 30 mM Tris-HCl with a pH of 8.3, 0.5 mM dNTP, and 70 mM (NH₄)₂SO₄. | |

Sample preparation: a DNA of non-small cell lung cancer EGFR exon 21 L861Q mutation was used as a positive sample, and the reverse primer (R) with a signal detection region sequence was used to detect the target sequence of non-small cell lung cancer EGFR exon 21 L861Q mutation, to verify a detection ability for single mutation. The ultra-pure water was used as a no template control (NTC).

### Reaction preparation:

After the sample preparation was completed, the reaction system was prepared according to the ratios described in Table 15.

### PCR amplification and melting curve analysis:

After a PCR tube was sealed by a cap, the sample was gently and uniformly mixed, and centrifuged for a short time and then left for 5 min at a room temperature. The PCR tube was put in a handheld centrifuge again, centrifuged for a short time, and then transferred to a tray of SLAN real-time fluorescence quantitative PCR instrument from Shanghai Hongshi Medical Technology Co., Ltd. The procedure used was as follows: performing pre-denaturation at 95°C for 3 minutes; performing denaturation at 94 °C for 10 seconds, and performing annealing and extension at 56 °C for 30 seconds, for a total of 45 cycles, without lighting; performing denaturation at 94 °C for 150 seconds, and perfroming incubation at a constant temperature of 40°C for 300 seconds; and performing melting curve analysis at 62-86°C, with a heating rate of 0.05°C/s and with lighting, to obtain a melting curve, as shown in Fig. 17.

### Data analysis:

The results were interpreted via a Tm value of the melting curve by using an analysis software of the SLAN real-time fluorescence quantitative PCR instrument from Shanghai Hongshi Medical Technology Co., Ltd.

Fig. 17 shows that a melting peak of non-small cell lung cancer EGFR exon 21 L861Q mutation appears in a PCR reaction well with addition of a non-small cell lung cancer EGFR exon 21 L861Q mutation positive sample in an HEX channel. The height of the melting peak is relatively low, which may be related to the absence of the probe anchoring region in the forward primer (F8-1). It should be noted that, the examples described above are only used to illustrate the present invention, but do not constitute any limitation to the present invention. The present invention has been described with referrence to typical examples, but it should be understood that the words used therein are descriptive and explanatory vocabularies, rather than restrictive vocabularies. Modifications may be made to the present invention within the scope of the claims of the present invention, and revisions may be made without departing from the scope of the present invention. Although the present invention described herein relates to specific methods, materials, and examples, it does not mean that the present invention is limited to the specific examples disclosed herein. Instead, the present invention may be extended to all of the other methods and applications with the same functionality.

## Claims

1. A primer probe for PCR detection, comprising a first primer, a probe (P), and a second primer; and **characterized in that**,
the first primer comprises a target sequence binding region 1, which is a sequence that can specifically bind with a target sequence in a paired manner;
the probe (P) is a sequence that does not bind with any target sequence in a paired manner, and comprises a probe signal detection region (H); and the probe (P) is labeled with a detection label;
the second primer comprises a primer signal detection region (h) and a target sequence binding region 2; wherein, the target sequence binding region 2 is a sequence that can specifically bind with the target sequence in a paired manner, the primer signal detection region (h) is a sequence that does not bind with any target sequence in a paired manner, and a complementary sequence (h') of the primer signal detection region (h) specifically binds with the probe signal detection region (H) of the probe (P) in a paired manner; and the target sequence binding region 2 is located at 3' end of the primer signal detection region (h);
the first primer and the second primer specifically bind with the target respectively to produce a pre-amplification product, and the pre-amplification product contains one single-stranded pre-amplification product having the complementary sequence (h') of the primer signal detection region (h), the complementary sequence (h') in the single-stranded pre-amplification product specifically binds with the probe (P) and extends by ≥ 0 bases to form a double-stranded product, and the formation of the double-stranded product causes the probe to produce a detectable signal change.

2. The primer probe of claim 1, **characterized in that**, the first primer is a forward primer (F) and
the second primer is a reverse primer (R); and/or
the complementary sequence (h') of the primer signal detection region (h) is a forward-complementary sequence (h') of the primer signal detection region (h) or a reverse-complementary sequence (h') of the primer signal detection region (h); and/or
the complementary sequence (h') of the primer signal detection region (h) binds with the probe signal detection region (H) of the probe (P) in a forward-complementary paired manner or in a reverse-complementary paired manner; and/or
the double-stranded product is a secondary amplification double-stranded product;
preferably, the complementary sequence (h') of the primer signal detection region (h) is the reverse-complementary sequence (h') of the primer signal detection region (h); and/or
the reverse-complementary sequence (h') of the primer signal detection region (h) binds with the probe signal detection region (H) of the probe (P) in a reverse-complementary paired manner.

3. The primer probe of claim 1 or 2, **characterized in that**, the first primer further comprises a probe anchoring region (A); wherein, the probe anchoring region (A) is a sequence that does not bind with any target sequence in a paired manner, and is located at 5' end of the target sequence binding region 1; and
the probe (P) further comprises a primer anchoring region (A'); and the primer anchoring region (A') is a sequence complementary with the probe anchoring region (A) of the first primer.

4. The primer probe of any one of claims 1 to 3, **characterized in that**, the detection label comprises a first detection group and a second detection group, and the first detection group and the second detection group generate a signal change via a distance change; preferably, the first detection group is spaced from the second detection group by 3-250 angstroms; preferably, by 3-201 angstroms; more preferably, by 3-140 angstroms; and further preferably, the first detection group is a fluorescence reporter group, and the second detection group is a quencher group or other labeling groups that can produce a signal change with the first detection group via fluorescence resonance energy transfer.

5. The primer probe of claim 3 or 4, **characterized in that**, the probe (P) comprises 1-2 primer anchoring regions (A') and 1-2 probe signal detection regions (H);
preferably, the probe (P) is any one structure of 1) to 4);
1) the probe (P) comprising one primer anchoring region (A') and one probe signal detection region (H);
2) the probe (P) compring one probe signal detection region (H) and two primer anchoring regions (A'), wherein the two primer anchoring regions (A') are located on two sides of the probe signal detection region (H) respectively, and the two primer anchoring regions (A') are different in sequence;
3) the probe (P) comprising one primer anchoring region (A') and two probe signal detection regions (H), wherein the two probe signal detection regions (H) are located on two sides of the primer anchoring region (A'), and the two probe signal detection regions (H) are different in sequence; and
4) the probe (P) comprising two primer anchoring regions (A') and two probe signal detection regions (H), wherein the primer anchoring regions (A') and the probe signal detection regions (H) are arranged at intervals, the two primer anchoring regions (A') are different in sequence, and the two probe signal detection regions (H) are different in sequence.

6. The primer probe of claim 3 or 4, **characterized in that**, the primer anchoring region (A') of the probe (P) is located at 5' end of the probe signal detection region (H); and preferably, the detection groups on the probe (P) are labeled between the primer anchoring region (A') and the probe signal detection region (H).

7. The primer probe of claim 3 or 4, **characterized in that**, the primer anchoring region (A') of the probe (P) is located at 3' end of the probe signal detection region (H); and preferably, the detection groups on the probe (P) are labeled at 5' end of the probe signal detection region (H).

8. The primer probe of any one of claims 1 to 7, **characterized in that**, a blocking region is contained at 3' end of the probe (P).

9. The primer probe of any one of claims 1 to 8, **characterized in that**, the second primer contains an extention blocking region (M) at 5' end thereof;
and/or, the pre-amplification product contains one single-stranded pre-amplification product having the complementary sequence (h') of the primer signal detection region (h) and a complementary sequence (M') of the extension blocking region (M); and the complementary sequence (M') of the extention blocking region (M) is a sequence that does not specifically bind with any part of the probe (P) in a paired manner;
preferably, the complementary sequence (M') of the extention blocking region (M) is a forward-complementary sequence (M') of the extention blocking region (M) or a reverse-complementary sequence (M') of the extention blocking region (M); and/or
the complementary sequence (M') of the extention blocking region (M) is a sequence that does not bind with any part of the probe (P) in a forward-complementary paired manner or in a reverse-complementary paired manner;
more preferably, the complementary sequence (M') of the extention blocking region (M) is the reverse-complementary sequence (M') of the extention blocking region (M); and/or
the reverse-complementary sequence (M') of the extention blocking region (M) is a sequence that does not bind with any part of the probe (P) in a forward-complementary paired manner or in a reverse-complementary paired manner.

10. The primer probe of any one of claims 1 to 9, **characterized in that**, a Tm value of the primer signal detection region (h) is simultaneously lower than a Tm value of the target sequence binding region 1 in the first primer and a Tm value of the target sequence binding region 2 in the second primer.

11. The primer probe of claim 3, **characterized in that**, a Tm value of the primer anchoring region (A') is higher than the Tm value of the primer signal detection region (h).

12. A kit for PCR detection, comprising the primer probe for PCR detection of any one of claims 1 to 11; preferably, the kit for PCR detection further comprises an amplification reagent; further preferably, the amplification reagent comprises a DNA polymerase and dNTPs; and still further preferably, when a nucleic acid template is RNA, the amplification reagent further comprises a reverse transcriptase.

13. A primer probe set for PCR detection, **characterized by** comprising one probe (P), at least one first primer, and at least two second primers;
the first primer comprises a target sequence binding region 1, and different target sequence binding regions 1 are sequences that can specifically bind with different target sequences in a paired manner;
the probe (P) is a sequence that does not bind with any target sequence in a paired manner, and comprises a probe signal detection region (H); and the probe (P) is labeled with a detection label;
the at least two second primers are different from each other, each independently comprising a primer signal detection region (h) and a target sequence binding region 2; wherein, the target sequence binding regions 2 in different second primers are sequences that specifically bind with different target sequences in a paired manner, all of the primer signal detection regions (h) in different second primers are sequences that do not bind with any target sequence in a paired manner, and a complementary sequence (h') of the primer signal detection region (h) specifically binds with the probe signal detection region (H) of the probe (P) in a paired manner; the target sequence binding region 2 is located at 3' end of the primer signal detection region (h); and the primer signal detection regions (h) of different second primers are different from each other in sequence.

14. The primer probe set of claim 13, **characterized in that**, the first primer and the second primer specifically bind with the target respectively to produce a pre-amplification product, and the pre-amplification product contains one single-stranded pre-amplification product having a complementary sequence (h') of the primer signal detection region (h), the complementary sequence (h') in the single-stranded pre-amplification product specifically binds with the probe (P) and extends by ≥ 0 bases to form a double-stranded product, and the formation of the double-stranded product causes the probe to produce a signal change; and/or
the single-stranded pre-amplification products formed by different second primers are different, and the double-stranded products formed by the binding of different single-stranded pre-amplification products with the probe (P) are different, and Tm values of different double-stranded products are different; and/or
the first primer is a forward primer (F) and the second primer is a reverse primer (R); and/or
the complementary sequence (h') of the primer signal detection region (h) is a forward-complementary sequence (h') of the primer signal detection region (h) or a reverse-complementary sequence (h') of the primer signal detection region (h); and/or
the complementary sequence (h') of the primer signal detection region (h) binds with the probe signal detection region (H) of the probe (P) in a forward-complementary paired manner or in a reverse-complementary paired manner; and/or
the double-stranded product is a secondary amplification double-stranded product;
preferably, the complementary sequence (h') of the primer signal detection region (h) is the reverse-complementary sequence (h') of the primer signal detection region (h); and/or
the reverse-complementary sequence (h') of the primer signal detection region (h) binds with the probe signal detection region (H) of the probe (P) in a reverse-complementary paired manner or in a forward-complementary paired manner.

15. The primer probe set of claim 14, **characterized in that**, at least one of the first primer further comprises a probe anchoring region (A); wherein the probe anchoring region (A) is a sequence that does not bind with any target sequence in a paired manner, and is located at 5' end of the target sequence binding region 1; and
the probe (P) further comprises a primer anchoring region (A'); and the primer anchoring region (A') is a sequence complementary with the probe anchoring region (A) of the first primer.

16. The primer probe set of claim 14 or 15, **characterized in that**, the detection label comprises a first detection group and a second detection group, and the first detection group and the second detection group generate a signal change via a distance change; preferably, the first detection group is spaced from the second detection group by 3-250 angstroms; preferably, by 3-201 angstroms; more preferably, by 3-140 angstroms; and further preferably, the first detection group is a fluorescence reporter group, and the second detection group is a quencher group or other labeling groups that can produce a signal change with the first detection group via fluorescence resonance energy transfer.

17. The primer probe set of any one of claims 13 to 16, **characterized in that**, the probe (P) comprises 1-2 primer anchoring regions (A') and 1-2 probe signal detection regions (H);
preferably, the probe (P) is any one structure of 1) to 4);
1) the probe (P) comprising one primer anchoring region (A') and one probe signal detection region (H);
2) the probe (P) compring one probe signal detection region (H) and two primer anchoring regions (A'), wherein the two primer anchoring regions (A') are located on two sides of the probe signal detection region (H), respectively, and the two primer anchoring regions (A') are different in sequence;
3) the probe (P) comprising one primer anchoring region (A') and two probe signal detection regions (H), wherein the two probe signal detection regions (H) are located on two sides of the primer anchoring region (A'), and the two probe signal detection regions (H) are different in sequence; and
4) the probe (P) comprising two primer anchoring regions (A') and two probe signal detection regions (H), wherein the primer anchoring regions (A') and the probe signal detection regions (H) are arranged at intervals, the two primer anchoring regions (A') are different in sequence, and the two probe signal detection regions (H) are different in sequence.

18. The primer probe set of any one of claims 12 to 15, **characterized in that**, a blocking region is contained at 3' end of the probe (P).

19. The primer probe set of any one of claims 13 to 18, **characterized in that**, an extention blocking region (M) is contained at 5' end of at least one of the second primer,
and/or, the pre-amplification product contains one single-stranded pre-amplification product having a complementary sequence (h') of the primer signal detection region (h) and a complementary sequence (M') of a extension blocking region (M); and the complementary sequence (M') of the extention blocking region (M) is a sequence that does not specifically bind with any part of the probe (P) in a paired manner;
preferably, the complementary sequence (M') of the extention blocking region (M) is a forward-complementary sequence (M') of the extention blocking region (M) or a reverse-complementary sequence (M') of the extention blocking region (M); and/or
the complementary sequence (M') of the extention blocking region (M) is a sequence that does not bind with any part of the probe (P) in a forward-complementary paired manner or in a reverse-complementary paired manner; and
more preferably, the complementary sequence (M') of the extention blocking region (M) is the reverse-complementary sequence (M') of the extention blocking region (M).

20. The primer probe set of any one of claims 13 to 19, **characterized in that**, a Tm value of the primer signal detection region (h) is simultaneously lower than a Tm value of the target sequence binding region 1 in the first primer and a Tm value of the target sequence binding region 2 in the second primer.

21. The primer probe set of any one of claims 13 to 20, **characterized in that**, a Tm value of the primer anchoring region (A') is higher than the Tm value of the primer signal detection region (h).

22. A kit for PCR detection, comprising the primer probe set for PCR detection of any one of claims 13 to 21, preferably, the kit for PCR detection further comprises an amplification reagent; further preferably, the amplification reagent comprises a DNA polymerase and dNTPs; and still further preferably, when a nucleic acid template is RNA, the amplification reagent further comprises a reverse transcriptase.

23. A primer probe composition for PCR detection, comprising at least one primer probe set of any one of claims 12 to 19.

24. The primer probe composition of claim 23, **characterized in that**, the probes (P) of different primer probe sets are different, the probes (P) of different primer probe sets are different in sequence, and preferably, the probes (P) of different primer probe sets are different in sequence and detection label.

25. Use of the primer probe composition for PCR detection of claim 23 or 24 in preparation of reagents for detecting various different targets.

26. A kit for PCR detection, comprising the primer probe composition for PCR detection of claim 23 or 24, and preferably further comprising an amplification reagent; further preferably, the amplification reagent comprises a DNA polymerase and dNTPs; and still further preferably, when a nucleic acid template is RNA, the amplification reagent further comprises a reverse transcriptase.

27. A method for performing PCR detection to a sample to be detected by using the kit of any one of claims 12, 22, and 26, comprising the following steps:
S1, mixing the first primer, the second primer, and the probe (P) with the amplification reagent and the sample to be detected, to obtain a reaction system;
S2, performing PCR amplification to the reaction system; and
S3, analyzing whether there is a target.

28. The method of claim 27, **characterized in that**, the step S3 comprises the following steps:
S3-1, obtaining a signal change generated by the PCR amplification in step S2, and judging whether there is a target in the sample to be detected based on the signal change; and/or
S3-2, heating a product obtained by the PCR amplification in step S2, and judging whether there is a target contained in the sample to be detected based on a signal change.

29. The method of claim 28, **characterized in that**, the step S2 comprises the following steps:
S2-1, randomly allocating the reaction system into 500 or more reaction units, wherein each reaction unit contains one target of the sample to be detected or does not contain a target of the sample to be detected; and/or,
S2-2, performing PCR amplification to all of the reaction units;
and/or, the step S3 comprises the following steps:
S3, obtaining a signal situation of each reaction unit after the PCR amplification in step S2, and judging whether there is a target based on presence or absence of a signal.

30. A method for performing PCR detection to a sample to be detected by using the kit of any one of claims 12, 22, and 26, comprising the following steps:
allowing the first primer and the second primer to specifically bind with a target respectively, to produce, after amplification, a pre-amplification product containing the complementary sequence (h') of the primer signal detection region (h);
allowing the complementary sequence (h') of the primer signal detection region (h) to specifically bind with the probe (P) and extend to form a secondary amplification double-stranded product;
the formation of the double-stranded product causes the probe to generate a detectable signal change, and judging whether there is a target in the sample to be detected based on the signal change; and/or
heating the double-stranded product, wherein a melting of the double-stranded product causes the probe to produce a detectable signal change, and judging whether there is a target contained in the sample to be detected based on the signal change.

31. The method of claim 28 or 30, **characterized in that**, when a Tm value at a peak position of the obtained melting curve is within a range of a characteristic Tm value of the target in the sample to be detected in a specific detection channel, it indicates that there is a target contained in the sample to be detected; and preferably, the specific detection channel is a fluorescent channel corresponding to the first detection group on the probe (P), and the characteristic Tm value is a melting temperature of the secondary amplification double-stranded product formed with the probe (P).
